# EUROPÄISCHE PATENTSCHRIFT

(11) **EP 1 731 587 B1**
(45) Veröffentlichungstag und Bekanntmachung des Hinweises auf die Patenterteilung: **23.01.2013**
(21) Anmeldenummer: 06009285.5
(22) Anmeldetag: 05.05.2006
(51) Int. Cl.: C07C 39/21, C07C 39/23, C07C 43/253, C07C 43/23, C07C 69/92, C07C 309/66, C09K 19/14, C09K 19/18

(54) **Verfahren zur Herstellung von Tolanen**
Process for the preparation of tolanes
Procédé de préparation de tolanes

(30) Priorität: 03.06.2005 DE 102005025973
(43) Veröffentlichungstag der Anmeldung: 13.12.2006
(73) Patentinhaber: Merck Patent GmbH, 64293 Darmstadt (DE)
(72) Erfinder: Wächtler, Andreas, Dr., 72076 Tübingen (DE); Wagner, Robert, Dr., 64807 Dieburg (DE); Stumpf, Hans-Bernd, 64646 Heppenheim (DE); Müller, Sebastian, 64750 Lützelbach (DE); Becker, Sylvia, 64291 Darmstadt (DE)

(56) Entgegenhaltungen:
- EP-A- 0 113 213
- EP-A- 0 648 723
- EP-A- 1 052 238
- WO-A-88/07514
- WO-A-92/18448
- DE-A1- 2 309 501
- DE-A1- 10 064 291
- DE-A1- 10 120 024
- DE-A1- 19 907 941
- US-A- 3 928 399
- US-A1- 2003 011 725
- NEWMAN, M.S. ET AL.: "The synthesis of diarylacetylenes" J. ORG. CHEM., Bd. 23, 1958, Seiten 665-666, XP002401931
- KOTLYAREVSKII, I.L. ET AL.: "Synthesis of certain substituted triphenylcarbinols" BULL. ACAD. SCI. USSR DIV. CHEM. SCI. (ENGL. TRANSL.), Bd. 16, Nr. 1, 1967, Seiten 205-207, XP009073075
- KAMIKAWA, T. ET AL.: "Dichloro[(2-dimethylamino)propyldiphenylp hosphine]palladium(II)(PdCl2(alaphos)): An efficient catalyst for cross-coupling of aryl triflates with alkynyl Grignard reagents" J. ORG. CHEM., Bd. 63, Nr. 24, 1998, Seiten 8922-8925, XP002401932
- DATABASE CAPLUS [Online] CHEMICAL ABSTRACTS SERVICE, COLUMBUS, OHIO, US; FUJIMOTO, YUKARI ET AL: "Preparation of tolan compound" XP002401933 gefunden im STN Database accession no. 1996:191704 -& JP 08 012599 A (SUMITOMO CHEMICAL CO., LTD., JAPAN) 16. Januar 1996 (1996-01-16)
- PATENT ABSTRACTS OF JAPAN Bd. 2000, Nr. 13, 5. Februar 2001 (2001-02-05) -& JP 2000 281603 A (SUMITOMO CHEM CO LTD; AGENCY OF IND SCIENCE & TECHNOL; NEW ENERGY & IN), 10. Oktober 2000 (2000-10-10)
- SEKINE, C. ET AL.: "Synthesis and properties of some novel high birefringence phenylacetylene liquid crystal materials with lateral substituents" LIQUID CRYSTALS, Bd. 28, Nr. 9, 2001, Seiten 1375-1387, XP009073283
- SEKINE C ET AL: "HIGH BIREFRINGENCE PHOTOPOLYMERIZABLE PHENYLACETYLENE LIQUID CRYSTALS" LIQUID CRYSTALS, TAYLOR AND FRANCIS, ABINGDON, GB, Bd. 28, Nr. 10, Oktober 2001 (2001-10), Seiten 1505-1512, XP001117381 ISSN: 0267-8292
- DATABASE WPI Week 199116 Derwent Publications Ltd., London, GB; AN 1991-112639 XP002401937 & JP 03 052837 A (ADEKA ARGUS CHEM CO LTD) 7. März 1991 (1991-03-07)
- GODT A. ET AL: "EPR probes with well-defined, long distances between two or three unpaired electrons", J. ORG. CHEM., vol. 65, 2000, pages 7575-7582,
- EICHHORN S.H. ET AL: "The interplay of bent-shape, lateral dipole and chirality in thiophene based di-, tri-, and tetracatenar liquid crystals", J. AM. CHEM. SOC., vol. 124, 2002, pages 12742-12751,
- OHGIYA T. ET AL: "A simple deprotection of triflate esters of phenol derivatives", TETRAHEDRON LETTERS, vol. 45, 2004, pages 6317-6320,

## Beschreibung

Die vorliegende Erfindung betrifft Verfahren zur Herstellung der Tolane der allgemeinen Formel I 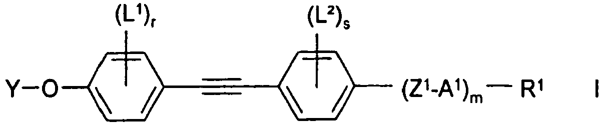 worin
- Y: eine Alkylsulfonyl-, Perfluoralkylsulfonyl-, Arylsulfonyl- oder Heteroarylsulfonyl-Schutzgruppe,
- L¹, L²: unabhängig voneinander, gleich oder verschieden F, Cl, eine Alkyl- und/oder Alkoxy-Gruppe mit 1 bis 6 C-Atomen, in der ein oder mehrere H-Atome durch Fluor substituiert sein können,
- r, s: unabhängig voneinander, gleich oder verschieden 0, 1, 2 oder 3, vorzugsweise 0, 1 oder 2,
- Z¹: -C₂H₄-, -CH₂-CF₂-, -CF₂-CH₂-, -C₂F₄-, -CH₂-O-, -O-CH₂-, -CF₂-O-, -O-CF₂-, -C≡C-, -O-C(O)-, -C(O)-O- oder eine Einfachbindung,
- A¹: 1,4-Phenylen, worin eine oder mehrere CH-Gruppen durch N ersetzt sein können, 1,4-Cyclohexylen, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei diese Gruppen ein oder mehrfach mit Halogen, vorzugsweise F oder Cl, substituiert sein können,
- m: 0, 1 oder 2, und
- R¹: H, Halogen, CN, geradkettiges oder verzweigtes Alkyl mit 1 bis 25 C-Atomen, worin ein oder mehrere H-Atome durch Fluor substituiert sein können, und/oder worin eine oder mehrere nicht benachbarte -CH₂- Gruppen unabhängig voneinander durch -O-, -C≡C- oder -CH=CH- ersetzt sein können, bedeuten,
dadurch gekennzeichnet, dass man eine Verbindung der Formel II 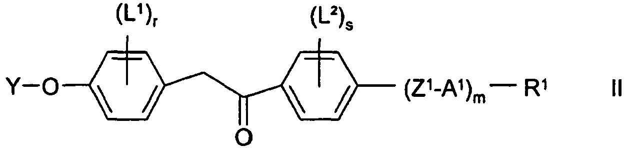 und/oder eine Verbindung der Formel III 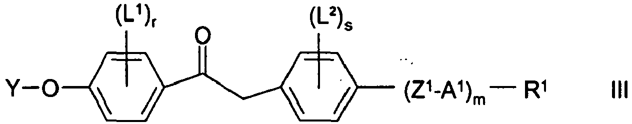 mit POCl₃ zusammen mit einer oder mehreren organischen Stickstoff-Basen umsetzt.

Eine Vielzahl in 4- und 4'-Position substituierter Tolane, die gegebenenfalls zusätzlich an einer oder beiden Phenylen-Gruppen Fluor-Substituenten aufweisen, werden als flüssigkristalline Verbindungen in Flüssigkristallmischungen für optische Anzeigeelemente, wie z.B. TN-, STN- und TFT-Displays, verwendet.

Aus der US 3,928,399 sind 4-Methoxy-4'-Hydroxytolanester der Formel 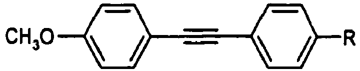 worin R -O-CO-CₙH₂ₙ₊₁ oder -O-CO-O-CₙH₂ₙ₊₁ mit n 1 bis 10 bedeutet, als nematische Flüssigkristalle bekannt. Die Synthese erfolgt unter anderem über die entsprechenden mit R in 4'-Position substituierten 4-Hydroxytolane.

In der JP 03-52837 A werden Isopentenyloxytolane der Formel 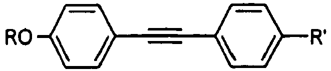 in der R 3-Methyl-2-butenyl oder 3-Methyl-3-butenyl und R' Alkyl mit 1 bis 18 C-Atomen bedeutet, zum Einsatz in elektrooptischen Displays und als Zwischenprodukte zur Synthese beschrieben. Die Herstellung dieser Verbindungen erfolgt über die 4-Alkyl-4'-hydroxytolane und die entsprechenden Buten-1-ole.

Die JP 02-231454 A betrifft chirale, flüssigkristalline Verbindungen für optische Anzeigeelemente. Dabei werden unter anderem Tolane der folgenden Formel offenbart 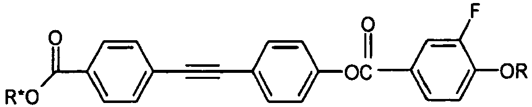 worin R* eine optisch aktive Gruppe und R n-Alkyl mit 1 bis 20 C-Atomen bedeutet. Die Synthese erfolgt durch Umsetzung eines Arylbromids mit einer Acetylenverbindung.

Die JP 04-159241 A betrifft ebenfalls optisch aktive Acetylen-Verbindungen zum Einsatz in Flüssigkristallmischungen, die durch die folgende Formel beschrieben werden

R¹-A-C≡C-B-(CH₂)ₙ-O-CH₂-C*H(X)-R²

in der R¹ C₆₋₁₅-Alkyl oder -Alkoxy, R² C₄₋₁₀-Alkyl, n 0 oder 1 und X Halogen sowie A und B Phenylen, Fluorphenylen oder Pyrimidinylen bedeuten. Der Aufbau des Tolan-Grundgerüsts erfolgt durch Reaktion einer AcetylenVerbindung mit einem Aryliodid in Gegenwart von Kupferiodid und einem Palladium-Katalysator.

In der JP 11/012228 werden Hydroxytolane nach folgendem Schema über Alkin-Arylhalogenid Kopplung hergestellt:

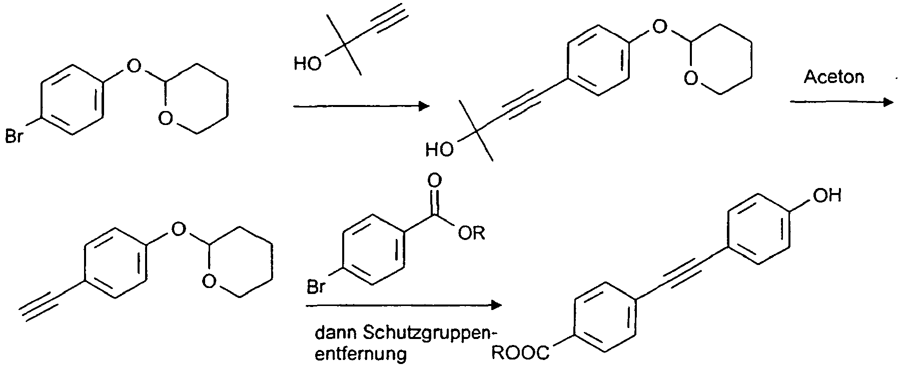

In der WO 88/07514 A1 werden Tolane der allgemeinen Formel

R¹-(A¹-Z¹)ₘ-A³-C≡C-A⁴-(A²)ₙ-R²

offenbart, worin A¹, A², A³, A⁴, R¹, R², Z¹, m und n die in diesem Dokument angegebenen Bedeutungen aufweisen. Zur Synthese dieser Tolane werden mehrere Varianten angeführt. So können, ausgehend von den entsprechenden Stilbenen, diese bromiert und anschließend dehydrohalogeniert werden. Ferner kann von Verbindungen ausgegangen werden, die an Stelle der -C≡C-Bindung eine -CH₂-CO-Gruppe enthalten. Diese werden mit einem anorganischen Säurechlorid umgesetzt und die dann entstandene -CH₂-CCl₂-Gruppe in Gegenwart einer Base dehydrohalogeniert. Vorzugsweise werden die Tolane durch Umsetzung aus substituierten Phenylacetylenen und Arylhalogeniden in Gegenwart eines Palladium-Katalysators und Kupfer(I)-iodid erhalten.

Fluortolane der folgenden Formel werden in der WO 92/18448 A1 offenbart 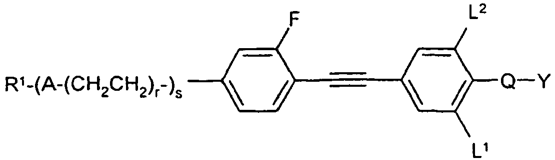 worin A, L¹, L², R¹, Q, Y, r und s die in diesem Dokument angegebenen Bedeutungen aufweisen. Als Synthesemethoden werden die bereits in der WO 88/07514 A1 offenbarten Verfahren beschrieben.

Ein weiteres Verfahren zur Herstellung von zwei- oder mehrfach substituierten Tolanen wird in der DE 41 05 743 C1 beschrieben. Danach wird ein substituiertes Benzylarylsulfon in Gegenwart eines Kondensationsmittels mit einem substituierten Benzaldehyd umgesetzt und das erhaltene Arylsulfonstilben-Derivat mit einer starken Base behandelt.

Ein für die Synthese von Tolanen über die Kreuzkopplung von Aryltriflaten mit Alkinyl-Grignard-Reagenzien geeigneter Palladium-Katalysator wird in dem Artikel von T. Kamikawa und T. Hayashi (J. Org. Chem. 63 (1998), 8922-8925) untersucht. Im Gegensatz zur Sonogashira-Reaktion im engeren Sinn, bei der Arylhalogenide mit Acetylen-Verbindungen in Gegenwart eines Palladium-Katalysators und Kupfer(I)-iodid umgesetzt werden, erfolgt die Kopplung auch bei 4-Bromphenyltriflat chemoselektiv an der Triflat-Gruppe.

Die in dem oben angeführten Stand der Technik zur Synthese von Tolanen beschriebene Sonogashira-Reaktion führt in der Regel nur mit Aryliodiden in Bezug auf das eingesetzte terminale Acetylen zu befriedigenden Ausbeuten, da beispielsweise bei den Bromiden oder perfluorierten Sulfonaten in erheblichem Ausmaß eine konkurrierende Homokopplung des terminalen Acetylens zu einem Diacetylen erfolgt. Die Folge davon ist, dass das terminale Acetylen im Überschuß eingesetzt werden muß. Aryliodide sind teurer als die entsprechenden Arylbromide, die bei der Umsetzung jedoch einen hohen Überschuss an der Acetylenverbindung erfordern. Daher ist der Einsatz der kostengünstigeren Arylbromide nur bei ebenfalls kostengünstigen Acetylenverbindungen gerechtfertigt. Jedoch sind die für die Synthese von flüssigkristallinen Tolanen interessanten p-substituierten Phenylacetylene nur mit sehr großem Syntheseaufwand zugänglich. Darüber hinaus neigen viele dieser Acetylene dazu, sich bei Temperaturen oberhalb von 120°C unter Freisetzung von Wärme zu zersetzen, was im großem Maßstab bei der Sonogashira-Reaktion zu einem Sicherheitsrisiko führt.

In den folgenden Druckschriften werden bereits substituierte Tolane offenbart, die in der Position des Gruppe -O-Y nach Formel I eine Trifluormethylsulfonylgruppe tragen: a) Kamikawa et al., J. Org. Chem. (1998) 63, 8922-8925, b) JP 08-012599 A, c) DE 10120024 A1, d) US 2003/011725, e) JP 2000-281603 A, f) DE 19907941 A1, g) Sekine et al., Liquid Crystals (2001) 28, 1375-1387, g) Sekine et al., Liquid Crystals (2001) 28, 1505-1512, h) DE 10064291 A1. Die zitierten Verbindungen werden gemäß den Zitaten durch Kupplung von Phenylalkinen mit Halogenbenzolderivaten oder aus den entsprechenden Hydroxytolanen hergestellt.

Ausgehend von dem genannten Stand der Technik kann es als Aufgabe der vorliegenden Erfindung angesehen werden, ein Verfahren zur Herstellung von Tolanen der Formel I bereitzustellen, bei dem leicht zugängliche Ausgangsverbindungen eingesetzt werden, das hohe Ausbeuten bei nur geringer Nebenproduktbildung liefert und das von der Reaktionsführung eine kostengünstige Herstellung in kommerziellem Maßstab erlaubt.

Gegenstand der Erfindung ist demnach ein Verfahren zur Herstellung von Tolanen der Formel I, das dadurch gekennzeichnet ist, dass man eine Verbindung der Formel II 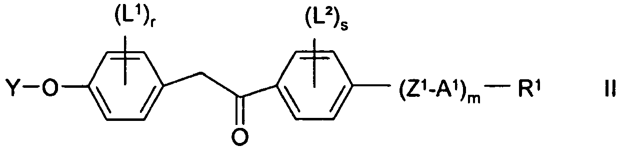 und/oder eine Verbindung der Formel III 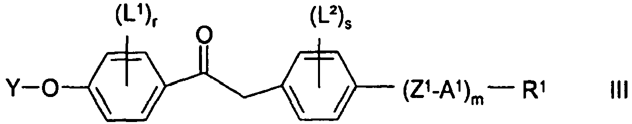 worin Y, L¹, L², Z¹, A¹, R¹, r, s und m die in bezug auf Formel I angegebenen Bedeutungen aufweisen,
mit PCl₃ und einer oder mehreren organischen Stickstoff-Basen umsetzt.

Ein besonderer Vorteil des erfindungsgemäßen Verfahrens ist die Umsetzung der Benzylphenylketone der Formel II und/oder III als Ausgangsverbindungen in nur einem Syntheseschritt in die entsprechenden Tolane. Völlig unerwartet ist hierbei die beobachtete Stabilität der Schutzgruppe Y während der Umsetzung. Überraschend sind daher insbesondere die erzielten, guten bis sehr guten Ausbeuten bei nur geringer Nebenproduktbildung. Zudem sind die Ausgangsverbindungen ebenfalls leicht zugänglich, so dass das erfindungsgemäße Verfahren eine kostengünstige Herstellung dieser Tolane in kommerziellem Maßstab erlaubt.

Die nach dem erfindungsgemäßen Verfahren erhältlichen Tolane der Formel I können prinzipiell auch durch Sonogashira Reaktion hergestellt werden, sofern Y nicht Perfluoralkylsulfonyl ist. Diese Tolane stellen für die Synthese von flüssigkristallinen Tolanen und polymerisierbaren, flüssigkristallinen Tolanen wichtige Ausgangsverbindungen dar. Zur Ankopplung von weiteren gegebenenfalls substituierten Aryl-, Heteroaryl- oder Alkylgruppen, insbesondere über eine Ether- oder Esterbildung, ist die über die Abspaltung der Sulfonyl-Schutzgruppe leicht zugängliche Hydroxyl-Gruppe von besonderem Vorteil. Weiterhin sind Perfluoralkylsulfonate, beispielsweise Triflate oder Nonafluorbutansulfonate, über die freie Hydroxylgruppe leicht zugänglich. Diese können bei metallkatalysierten Kreuzkopplungen, zum Beispiel der Suzuki-Kopplung, der Heck-Reaktion oder der Sonogashira-Kopplung, anstelle der entsprechenden Halogen-Derivate eingesetzt werden. Zwar ist die freie Hydroxyl-Gruppe auch aus den bekannten Alkyltolanethern zugänglich, jedoch ist die damit verbundene Etherspaltung von Nachteil. So werden beispielsweise bei der Spaltung der entsprechenden Methyl-tolanether toxische Alkylierungsmittel, wie z.B. Methylhalogenide, freigesetzt oder toxische und geruchsbelästigende Schwefelverbindungen eingesetzt, wenn man die Etherspaltung mit Thiolaten durchführt.

Ferner werden die im erfindungsgemäßen Verfahren als Ausgangsverbindungen einsetzbaren Benzylphenylketone sowohl der Formel II als auch der Formel III offenbart. Diese Benzylphenylketone unterscheiden sich von den Tolanen der Formel I lediglich in der -CH₂-CO- bzw. -CO-CH₂- Gruppe an Stelle der Ethinylen-Brücke.

Ein Verfahren zur Herstellung der Benzylphenylketone der Formel II ist die Friedel-Crafts-Acylierung von Verbindungen der Formel V mit Säurechloriden der Formel IV in Gegenwart eines Friedel-Crafts Katalysators 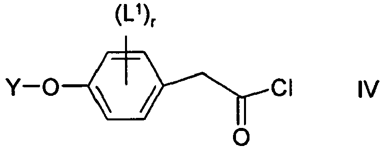 worin Y eine Sulfonyl-Schutzgruppe ist. 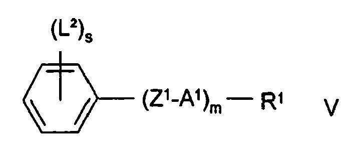 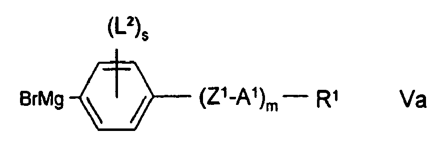

Entgegen den Erwartungen hat sich völlig überraschend gezeigt, dass die Friedel-Crafts-Acylierung bei Y gleich Acyl praktisch ohne konkurrierende Fries-Verschiebung zu dem gewünschten Benzylphenylketon verläuft und dass die erwarteten Fries-analogen Umlagerungen bei Y gleich Sulfonyl bzw. Oxycarbonyl ebenfalls praktisch nicht auftreten.

Ketone der Formel II sind weiterhin leicht zugänglich, indem man eine Grignard-Verbindung des Typs Va mit einem Säurechlorid IV in Gegenwart katalytischer Mengen an Fe(acac)₃ unter den Bedingungen von V. Finandese, G. Marchese, V. Martina und L. Ronzini, THL 25, 4805-4808 (1984) umsetzt. Diese Methode ist deswegen besonders nützlich, weil die Grignard-Verbindungen, soweit es die Substituenten (-Z¹-A¹)ₘ-R¹) zulassen, klassisch aus dem entsprechenden Arylbromid und Magnesium herstellbar sind oder andernfalls durch Umgrignardierung der entsprechenden Aryliodide (nach Knochel et al., Angew. Chem. 2003, 115, 4438-4456) bzw. der entsprechenden Arylhalogenide (nach Krasovskiy und Knochel, Angew. Chem. 2004, 116, 3396) mit iso-PropylMgCl bzw. iso-PropylMgCl/LiCl erhalten werden können. Diese Umgrignardierung nach Knochel erlaubt die Herstellung von Grignardverbindungen der Formel Va, bei denen die Substituenten (Z¹-A¹)ₘ-R¹ auch Estergruppen enthalten können. Grignardverbindungen, die direkt beispielsweise aus Arylbromid und Magnesium herstellbar sind, und keine empfindlichen Substituenten tragen, kann man auch nach der Ketonsynthese von Weinreb mit den von den Säurechloriden IV abgeleiteten N-Methoxy-N-methylamiden zu den Ketonen der Formel II umsetzen.

Darüber hinaus kann man die Ketone der Formel II aus den entsprechenden Säurechloriden IV und den lithiumanalogen Verbindungen der Grignard-Verbindungen Va erhalten, wenn man zuvor diese lithiumorganischen Verbindungen beispielsweise mit MnJ₂ in die entsprechenden manganorganischen Verbindungen (nach Knochel et al., THL 38, 1927-30 (1997)) überführt. Geeignete Lithiumverbindungen sind durch Halogen-Metallaustausch der entsprechenden Arylhalogenide oder durch Orthometallierung zugänglich.

Knochel hat gezeigt, daß man organische Sulfonsäureester von ortho-Jodphenolen bei tiefen Temperaturen ebenfalls, beispielsweise mit iso-Propylmagnesiumchlorid, umgrignardieren kann, wobei sich beim Erwärmen auf etwa -15°C Arine bilden (Knochel et al., Angew. Chem. Int. Ed., 43, 4364 (2004)). Bei den Sulfonsäureestern von para-Halogenphenolen ist diese Arinbildung nicht möglich.

Durch das erfindungsgemäße Verfahren sind beispielsweise die folgenden Verbindungen erhältlich (Tos = Toluolsulfonyl, Mes = Methansulfonyl):

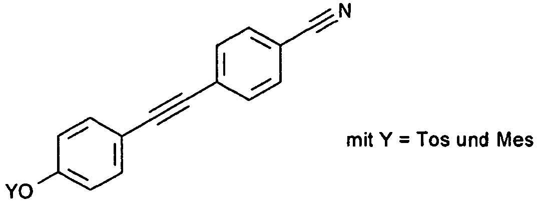

Ein weiterer Gegenstand der Offenbarung ist die Verwendung der Tolane der Formel I zur Herstellung von flüssigkristallinen Tolanen der Formel IX 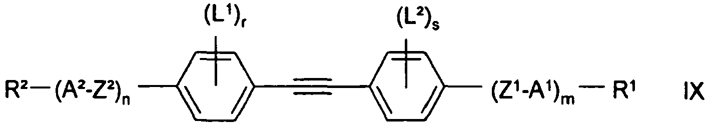 worin L¹, L², r, s, Z¹, A¹, m und R¹ die in Bezug auf Formel I angegebenen Bedeutungen aufweisen und
- Z²: -O-CO-, -CO-O-, -C₂H₄-, -CH₂-CF₂-, -CF₂-CH₂-, -C₂F₄-, -CH₂-O-, -O-CH₂-, -CF₂-O-, -O-CF₂-, -CH=CH-, -CF=CH-, -CH=CF-, -CF=CF-, -CH=CH-COO-, -OCO-CH=CH-, -CH=CF-COO-, -OCO-CH=CF-, -CF=CH-COO-, -OCO-CF=CH-, -CF=CF-COO-, -OCO-CF=CF-, -C≡C- oder eine Einfachbindung,
- A²: 1,4-Phenylen, worin eine oder mehrere CH-Gruppen durch N ersetzt sein können, 1,4-Cyclohexylen, worin eine oder zwei nicht benachbarte CH₂-Gruppen durch O und/oder S ersetzt sein können, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei diese Gruppen ein oder mehrfach mit F und/oder Cl substituiert sein können,
- n: 0, 1 oder 2, und
- R²: H, Halogen oder geradkettiges oder verzweigtes Alkyl mit 1 bis 25 C-Atomen, worin ein oder mehrere H-Atome durch Halogen substituiert sein können, und/oder worin eine oder mehrere nicht benachbarte -CH₂-Gruppen unabhängig voneinander durch -CO-, -O-CO-, -S-CO-, -CO-O-, -CO-S-, -O-, -S-, -CH=CH-, -CH=CF-, -CF=CH-, -CF=CF-, -C≡C-, -NH- oder -N(CH₃)- ersetzt sein können, bedeuten.

Tolane der Formel I werden zur Herstellung von polymerisierbaren, flüssigkristallinen Tolanen der Formel X verwendet: 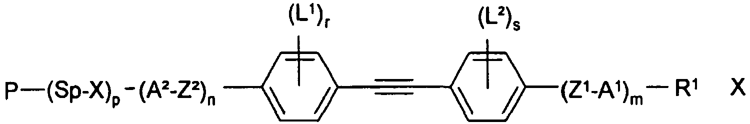 worin L¹, L², r, s, Z¹, A¹ und m sowie A², Z² und n die in Bezug auf die Formeln I und IX angegebenen Bedeutungen aufweisen,
- R¹: eine der in Bezug auf Formel I angegebenen Bedeutungen aufweist oder auch P-(Sp-X)p- bedeutet,
- P: CH₂=CW-COO-, WCH=CH-O-, 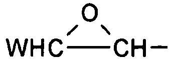 oder CH₂=CH-Phenyl-(O)ₖ-, worin W H, CH₃ oder Cl und k 0 oder 1 ist,
- Sp: eine Spacer-Gruppe mit 1 bis 25 C-Atomen,
- X: -O-, -S-, -CO-, -COO-, -OCO-, -CO-NH-, -NH-CO-, -CH₂CH₂-, -OCH₂-, -CH₂O-, -SCH₂-, -CH₂S-, -CH=CH-, -CH=CH-COO-, -OCO-CH=CH-, -C=C- oder eine Einfachbindung bedeuten, und
- p: 0 oder 1 ist.

Im folgenden werden vorteilhafte und bevorzugte Ausführungsformen des erfindungsgemäßen Verfahrens zur Herstellung von Tolanen der Formel I beschrieben.

Beim technischen Einsatz von flüssigem POCl₃ im Vergleich zum festen PCl₅ ist die leichtere Handhabung von Vorteil. Das Säurechlorid wird vorzugsweise im Molverhältnis von 1 : 1 bis 3 : 1, besonders bevorzugt von 1,5 : 1 bis 2,5 : 1 und insbesondere im Verhältnis 2 : 1 zu dem Edukt der Formel II und/oder der Formel III eingesetzt.

Als vorteilhaft hat sich die Verwendung von stickstoffhaltigen organischen Basen, insbesondere tertiären Aminen und/oder Pyridin, erwiesen. Beispiele für geeignete tertiäre Amine sind Triethylamin, Hünig-Base und analoge Verbindungen. Weitere geeignete Basen sind beispielsweise Picoline und 5-Ethyl-2-methylpyridin. Die Basen, insbesondere Pyridin, können vorzugsweise gleichzeitig als Lösungsmittel in dem Reaktionsmedium dienen. Vorzugsweise werden die Basen im Molverhältnis von 4 : 1 bis 20 : 1, besonders bevorzugt von 7 : 1 bis 10 : 1, zu dem Edukt der Formel II und/oder der Formel III eingesetzt.

Insbesondere bevorzugt ist der Einsatz von POCl₃ zusammen mit einem tertiären Amin oder Pyridin, im Hinblick auf eine hohe Ausbeute, eine geringe Nebenproduktbildung und eine gute technische Durchführbarkeit.

Das erfindungsgemäße Verfahren ausgehend von den Benzylphenylketonen der Formel II und/oder der Formel III, erfolgt besonders bevorzugt in einem Schritt, also ohne Isolierung und Aufreinigung etwaiger Zwischenprodukte. Hierzu werden das Säurechlorid und die Base vorzugsweise gleichzeitig eingesetzt. Die Umsetzung mit dem oder den Säurechloriden findet vorzugsweise also in Gegenwart der Base oder den Basen statt. Jedoch ist auch der zeitlich versetzte Einsatz des Säurechlorids und nach einer Reaktionszeit, vorzugsweise von 10 Minuten bis 3 Stunden, die Zugabe der Base denkbar.

Vorzugsweise wird die Base, insbesondere Pyridin, gleichzeitig als Lösungsmittel verwendet. Das Reaktionsmedium kann weitere Lösungsmittel aufweisen. Geeignete Lösungsmittel sind beispielsweise Toluol, Xylol und andere Aromaten. Das Verhältnis der Menge des Lösungsmittels zu dem Edukt ist nicht kritisch.

Die Umsetzung erfolgt vorzugsweise in einem Temperaturbereich von 20°C bis 160°C, besonders bevorzugt von 50°C bis 130°C und insbesondere im Bereich der Siedetemperatur des Reaktionsgemisches.

Die Reaktion kann in einem weiten Druckbereich, vorzugsweise in einem Druckbereich von 0,5 bis 5 bar, besonders bevorzugt bei Normaldruck, durchgeführt werden.

Je nach eingesetzten Edukten und Reaktionsparametern, wie Druck und Temperatur, liegt die Reaktionszeit vorzugsweise zwischen 15 Minuten und 48 Stunden, besonders bevorzugt zwischen 1 und 12 Stunden.

Die Aufarbeitung des Reaktionsgemisches erfolgt in einer dem Fachmann bekannten Weise. Gemäß einer für solche Reaktionen üblichen Variante wird gegebenenfalls das Reaktionsgemisch abgekühlt, Säure hinzugegeben, das ausgefallene Produkt abgetrennt, gewaschen, getrocknet und gegebenenfalls weiter aufgereinigt, beispielsweise durch ein- oder mehrfaches Umkristallisieren und/oder mittels chromatographischer Trennverfahren.

Die Alkyl-Gruppe in der Alkylsulfonyl-Schutzgrupppe kann dabei entweder eine geradkettige oder verzweigte Alkylgruppe sein, wie z.B.die Triisopropylsulfonyl-Gruppe. Vorzugsweise weist die Alkylgruppe 1 bis 6 Kohlenstoffatome auf. Bevorzugte Arylsulfonyl-Schutzgruppen sind die Phenylsulfonyl-, die Tosyl- oder die 2,4,6-Tris(iso-propyl)-phenylsulfonylGruppe. Bevorzugte Perfluoralkylsulfonyl-Schutzgruppen sind die Trifluormethansulfonyl- sowie die Nonafluorbutansulfonyl-Gruppe.

Es wurde beobachtet, dass die Stabilität der Schutzgruppen und die Ausbeute im erfindungsgemäßen Verfahren nach Anspruch 1 von Oxycarbonyl über Acyl zu Sulfonyl hin zunimmt.

Es werden daher Sulfonate als Ausgangsverbindungen eingesetzt. Die Schutzgruppe Y ist vorzugsweise ein Alkyl- oder

Arylsulfonyl, insbesondere ein Alkylsulfonyl der Formel 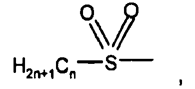 in der n 1 bis 6 ist, und daher Methyl-, Ethyl-, Propyl-, Butyl-, Pentyl- oder Hexyl-Sulfonyl, besonders bevorzugt Methyl-Sulfonyl bedeutet. In der Alkylsulfonyl- und Arylsulfonyl-Schutzgruppe können auch ein oder mehrere H-Atome durch Fluor substituiert sein.

Ausgehend von der Verbindung der Formel II und/oder der Formel III, worin Y jeweils H bedeutet, wird die Sulfonyl-Schutzgruppe vorzugsweise durch Umsetzung mit einem organischen Sulfonylhalogenid und/oder Sulfonylanhydrid erhalten.

Nachfolgend werden bevorzugte Ausführungsformen zur Herstellung der Benzylphenylketone der Formel II als Ausgangsverbindungen für das erfindungsgemäße Verfahren beschrieben.

Das Säurechlorid der Formel IV wird vorzugsweise in einem Molverhältnis von 1 : 0,9 bis 1 : 1,5, besonders bevorzugt von 1 : 1 bis 1 : 1,3, zu dem Edukt der Formel V eingesetzt.

Als Friedel-Crafts-Katalysator wird vorzugsweise Aluminiumchlorid verwendet. Weitere geeignete Acylierungskatalysatoren sind die üblichen Katalysatoren, wie sie beispielsweise im Standardwerk Houben-Weyl-Müller, Methoden der Organischen Chemie, Thieme-Verlag, Stuttgart beschrieben sind. Der Acylierungskatalysator wird vorzugsweise in einem Molverhältnis von 1 : 1 bis 4 : 1, vorzugsweise von 1,5 : 1 bis 2,3 : 1, zum Edukt der Formel V eingesetzt.

Als Lösungsmittel eignen sich vorzugsweise polare, inerte Lösungsmittel, insbesondere Halogenalkane, wie z.B. Dichlormethan, 1,2-Dichlorethan und andere für Friedel-Crafts Reaktionen üblicherweise verwendete Lösungsmittel, wie sie beispielsweise im genannten Standardwerk "Methoden der Organischen Chemie" beschrieben sind.

Die Umsetzung wird vorzugsweise in einem Temperaturbereich von -30°C bis 60°C, besonders bevorzugt von -20°C bis 40°C durchgeführt.

Die Reaktion kann in einem weiten Druckbereich, vorzugsweise in einem Druckbereich von 0,5 bis 5 bar, besonders bevorzugt bei Normaldruck, durchgeführt werden.

Besonders bevorzugt ist bei der Reaktionsführung folgende Vorgehensweise: In einer Apparatur wird unter Rühren das Aluminiumchlorid bei -5°C bis 20°C in einem geeigneten Lösungsmittel, wie z.B. Dichlormethan, suspendiert. Dazu gibt man langsam unter Temperaturkontrolle bei einer Temperatur von 5°C bis 35°C, vorzugsweise bei 10°C bis 20°C, eine Lösung von IV und V in Dichlormethan.

Je nach eingesetzten Edukten, Lösungsmitteln und verwendeten Reaktionsparametern, wie Temperatur und Druck, erfolgt die Umsetzung in einem Zeitraum von 15 Minuten bis 24 Stunden, vorzugsweise von 30 Minuten bis 12 Stunden.

Die Aufarbeitung des Reaktionsgemisches erfolgt in einer dem Fachmann bekannten Weise. Gemäß einer für solche Reaktionen üblichen Variante wird gegebenenfalls das Reaktionsgemisch abgekühlt, Säure hinzugegeben, die organische Phase abgetrennt, diese gewaschen, das Lösungsmittel abgetrennt und das Rohprodukt gegebenenfalls weiter aufgereinigt, beispielsweise durch ein- oder mehrfaches Umkristallisieren und/oder mittels chromatographischer Trennverfahren.

Das für die Synthese als Edukt erforderliche Säurechlorid der Formel IV kann nach bekannten Verfahren aus der entsprechenden Carbonsäure durch Umsetzung mit Thionylchlorid erhalten werden.

Wird als Ausgangsverbindung für die Carbonsäurechlorid-Synthese die leicht erhältliche p-Hydroxy-phenylessigsäure verwendet, so wird zunächst die Hydroxyl-Gruppe -OH durch Umwandlung in -OY nach bekannten Verfahren geschützt. So wird das Anbringen einer Sulfonylschutzgruppe, am Beispiel der Methylsulfonyl-Gruppe in Beispiel 1 der DE-OS 27 00 348 beschrieben. Die Synthese einer Methoxy- oder Ethoxycarbonyl-Schutzgruppe wird in Mol. Cryst. Liq. Cryst. 197, 21 (1991) sowie in JACS 701, 2837 (1948) beschrieben. Ferner sind die Verfahren für die Addition einer Acyl-Schutzgruppe, beispielsweise unter Verwendung von Acetanhydrid, dem Fachmann wohl bekannt.

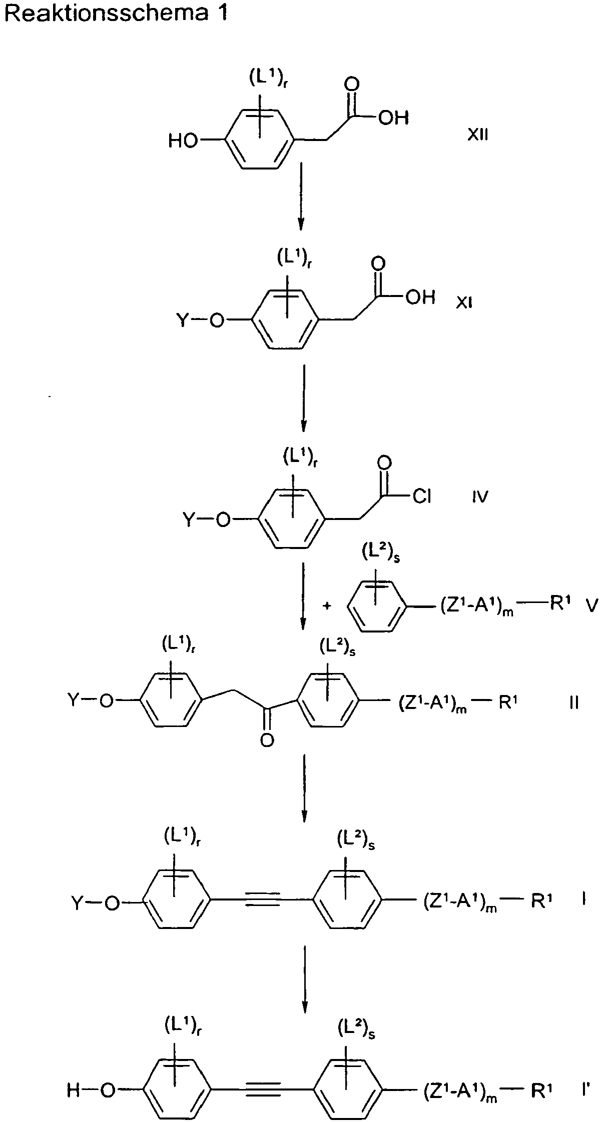

Gemäß obigem Reaktionsschema 1 sind mit den zuvor beschriebenen erfindungsgemäßen Verfahren die Tolane der Formel I ausgehend von p-Hydroxyphenylessigsäure der Formel XII, über die in para-Position mit der Schutzgruppe versehene Phenylessigsäure der Formel XI, das entsprechende Säurechlorid der Formel IV und das Produkt der Friedel-Crafts-Acylierung der Formel II erhältlich. Aus den Tolanen der Formel I sind die entsprechenden Hydroxytolane der Formel I' leicht erhältlich.

Zur Erhöhung der Ausbeute in dem erfindungsgemäßen Verfahren wird eine Sulfonyl-Schutzgruppe verwendet. Für den Fall, dass Y keine Sulfonyl-Schutzgruppe ist, wird hierzu aus dem Produkt II der Friedel-Crafts-Acylierung nach den genannten Verfahren die Schutzgruppe Y durch Hydrolyse alkalisch oder sauer abgespalten und durch eine Sulfonyl-Schutzgruppe ausgetauscht.

In den erfindungsgemäßen Verfahren und den Tolanen der Formel I hat Y als Sulfonyl- Schutzgruppe besonders bevorzugt folgende Bedeutung:

| Y | Formel |
|---|---|
| Sulfonyl- | 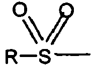 |

worin R ein geradkettiger oder verzweigter Alkylrest mit 1 bis 12 C-Atomen, in dem ein oder mehrere H-Atome durch Fluor substituiert sein können, oder ein gegebenenfalls substituierter Arylrest ist. Bevorzugte Bedeutungen für R sind Methyl, Ethyl, Propyl, Butyl, Pentyl, Hexyl, -CF₃, -C₂F₅, -C₃F₇, -C₄F₉, -C₅F₁₁, -C₆F₁₃, Phenyl oder Tolyl.

Die Benzylphenylketone der Formel III sind nach dem Fachmann bekannten Verfahren oder in Analogie dazu herstellbar.

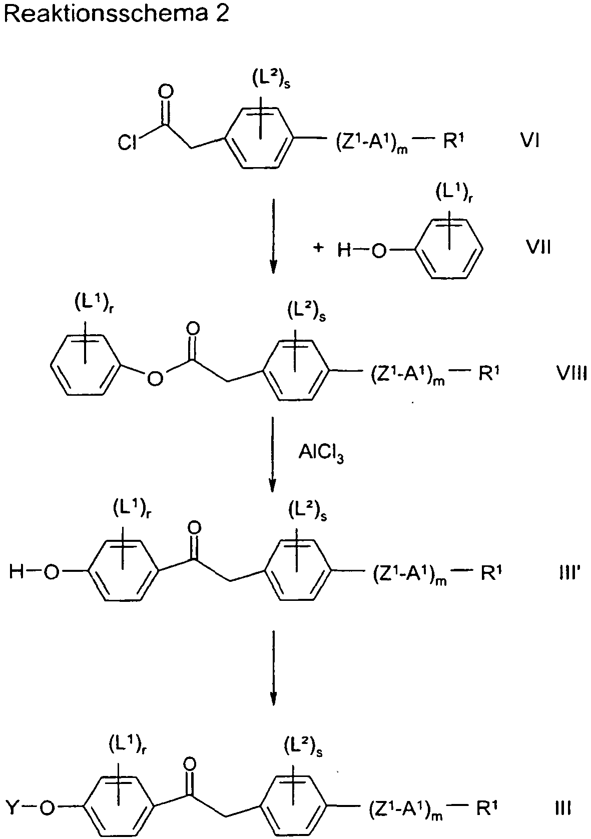

Zur Illustration wird im obigen Reaktionsschema 2 eine Synthesevariante beispielhaft dargestellt. Ausgehend von einer geeigneten, aktivierten Carbonsäure, hier als Säurechlorid der Formel VI, erfolgt eine Umsetzung mit einem gegebenenfalls substituierten Phenol der Formel VII zu der Esterverbindung der Formel VIII. Diese kann in Gegenwart eines geeigneten Katalysators, wie z.B. Aluminiumchlorid, unter dem Fachmann bekannten Bedingungen als sogenannte Fries-Umlagerung in das Benzylphenylketon der Formel III' mit freier Hydroxyl-Gruppe umgesetzt werden. Die gewünschten Verbindungen der Formel III mit der Schutzgruppe Y sind nach den zuvor angegebenen Verfahren ohne weiteres hieraus erhältlich. Die Synthese nach dieser Variante kann sowohl in zwei Stufen als auch in einer Stufe (in situ) durchgeführt werden.

Ein weiteres Verfahren zur Herstellung der Ketone der Formel III ist die Umsetzung der Säurechloride VI (Reaktionsschema 2) bei tiefen Temperaturen mit den Grignard-Verbindungen, die man durch Umgrignardierung von Sulfonsäureestern von para-Halogenphenolen erhält (wobei man bei Bromiden beispielsweise dem iso-Propylmagnesiumchlorid LiCl bei der Umgrignardierung zusetzt).

Die Benzylphenylketone der Formel II und III sind nicht nur besonders bevorzugt zur Synthese von Tolanen der Formel I geeignet, sondern stellen allgemein wertvolle Synthesebausteine dar. Die zwischen zwei gegebenenfalls substituierten Phenylgruppen vorhandene -CH₂-CO-Brücke ermöglicht die Umsetzung in Mannich-Reaktionen, Fischerschen Indolsynthesen, reduktiven Aminierungen und Knoevenagel-Reaktionen. Insbesondere mit einer Schutzgruppe Y = Sulfonyl ist auch der Einsatz sperriger Basen, wie z.B. tert.-Butylat, zur Enolat-Bildung möglich. Damit finden die Benzylphenylketone der Formel II und III sowohl zur Synthese von flüssigkristallinen Verbindungen als auch auf anderen Gebieten, wie z.B. zur Synthese von pharmazeutischen und kosmetischen Wirkstoffen, als auch zur Synthese von Farbstoffen und Pflanzenschutzmitteln, vielfältige Verwendungsmöglichkeiten.

Nachfolgend werden bevorzugte Bedeutungen der Gruppen und Substituenten der Tolane der Formel I, der Benzylphenylketone der Formel II und III, der flüssigkristallinen Tolane der Formel IX und der polymerisierbaren Tolane der Formel X aufgeführt.

Falls R¹ und/oder R² die Bedeutung Alkyl oder Alkoxy hat, kann der Alkyl- oder Alkoxy-Rest linear oder verzweigt sein. Vorzugsweise ist er linear und besitzt 2, 3, 4, 5, 6, 7 oder 8 C-Atome und bedeutet daher besonders bevorzugt Ethyl, Propyl, Butyl, Pentyl, Hexyl, Heptyl, Octyl, Ethoxy, Propoxy, Butoxy, Pentoxy, Hexoxy, Heptoxy oder Octoxy, des weiteren Methyl, Nonyl, Decyl, Undecyl, Dodecyl, Tridecyl, Tetradecyl, Pentadecyl, Methoxy, Nonoxy, Decoxy, Undecoxy, Dodecoxy, Tridecoxy oder Tetradecoxy.

Falls R¹ und/oder R² die Bedeutung Oxaalkyl hat, kann der Rest linear oder verzweigt sein. Vorzugsweise ist er linear und bedeutet beispielsweise 2-Oxapropyl (=Methoxymethyl), 2- (=Ethoxymethyl) oder 3-Oxabutyl (=2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl oder 2-, 3-, 4-, 5-, 6-, 7-, 8- oder 9-Oxadecyl.

R¹ und/oder R² können eine chirale oder achirale Gruppe sein. Besonders bevorzugt ist R¹ eine chirale Gruppe. Im Fall einer chiralen Gruppe entsprechen R¹ und/oder R² vorzugsweise folgender Formel XI 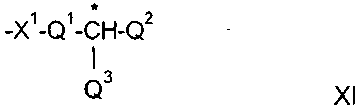 worin
- X¹: -O- oder eine Einfachbindung,
- Q¹: eine Alkylen- oder Alkylenoxy-Gruppe mit 1 bis 10 C-Atomen oder eine Einfachbindung,
- Q²: eine Alkyl- oder Alkoxy-Gruppe mit 1 bis 10 C-Atomen, in der ein oder mehrere H-Atome durch Fluor substituiert sein können, und/oder in der eine oder mehrere nicht benachbarte -CH₂-Gruppen unabhängig voneinander durch -C≡C- oder -O- derart ersetzt sein können, dass O-Atome nicht direkt miteinander verbunden sind, und
- Q³: Fluor, eine Cyano-Gruppe oder eine Alkyl- oder Alkoxy-Gruppe mit 1 bis 4 C-Atomen, die verschieden von Q² ist, bedeuten.

Im Falle von R² kann X¹ auch -O-, -S-, -CO-, -COO-, -OCO- oder -OCOO-, Q² auch eine Alkyl- oder Alkoxy-Gruppe mit 1 bis 10 C-Atomen, in der ein oder mehrere H-Atome auch durch Chlor substituiert sein können, und/oder in der ein oder mehrere nicht benachbarte CH₂-Gruppen unabhängig voneinander auch durch -O-, -S-, -NH-, -N(CH₃)-, -CO-, -COO-, -OCO-, -OCO-O-, -S-CO- oder -CO-S- derart ersetzt sein können, dass Heteroatome nicht direkt miteinander verbunden sind, und Q³ auch Chlor bedeuten.

Falls Q¹ gemäß Formel XI eine Alkylenoxy-Gruppe bedeutet, ist das O-Atom vorzugsweise benachbart zum chiralen C-Atom.

Bevorzugte chirale Gruppen R¹ und/oder R² sind 2-Butyl (=1-Methylpropyl), 2-Methylbutyl, 2-Methylpentyl, 3-Methylpentyl, 2-Ethylhexyl, 2-Propylpentyl, 2-Octyl, insbesondere 2-Methylbutyl, 2-Methylbutoxy, 2-Methylpentoxy, 3-Methylpentoxy, 2-Ethylhexoxy, 1-Methylhexoxy, 2-Octyloxy, 2-Oxa-3-methylbutyl, 3-Oxa-4-methylpentyl, 4-Methylhexyl, 2-Nonyl, 2-Decyl, 2-Dodecyl, 6-Methoxyoctoxy, 6-Methyloctoxy, 6-Methyloctanoyloxy, 5-Methylheptyloxycarbonyl, 2-Methylbutyryloxy, 3-Methylvaleroyloxy, 4-Methylhexanoyloxy, 2-Chlorpropionyloxy, 2-Chlor-3-methylbutyryloxy, 2-Chlor-4-methylvaleryloxy, 2-Chlor-3-methylvaleroyloxy, 2-Methyl-3-oxapentyl, 2-Methyl-3-oxahexyl, 1-Methoxypropyl-2-oxy, 1-Ethoxypropyl-2-oxy, 1-Propoxypropyl-2-oxy, 1-Butoxypropyl-2-oxy, 2-Fluorooctyloxy und 2-Fluorodecyloxy.

Die Synthese chiraler Gruppen R¹ und/oder R² und damit der entsprechenden chiralen Arylverbindungen der Formel V als Ausgangsverbindungen für die Friedel-Crafts-Acylierung ist entweder aus der Fachliteratur bekannt oder lässt sich in analoger Weise zu vergleichbaren, bekannten Umsetzungen durchführen. So sind beispielsweise vergleichbare Synthesen bekannt aus Basil Wakefield, Organomagnesium Methods in Organic Synthesis, Academic Press 1995, Seiten 169 ff. und dort zitierte Literatur sowie Chan, Gray, Lacey und Toyne, Mol. Cryst. Liq. Cryst. 158, 1988 (209-240).

Die erfindungsgemäßen Verfahren eignen sich daher besonders bevorzugt zur Synthese chiraler Friedel-Crafts-Acylierungsprodukte der Formel II sowie chiraler Tolane der Formel I. Über diese Tolane sind entsprechende chirale flüssigkristalline Tolane der Formel IX sowie chirale, polymerisierbare, flüssigkristalline Tolane der Formel X ebenso besonders bevorzugt zugänglich.

Auch Verbindungen mit einer achiralen Gruppe R¹ und/oder R² können von Bedeutung sein, beispielsweise zur Unterdrückung der Neigung zur Kristallisation. Verzweigte Gruppen diesen Typs enthalten vorzugsweise nicht mehr als eine Kettenverzweigung. Bevorzugte achirale verzweigte Gruppen sind Isopropyl, Isobutyl (=Methylpropyl), Isopentyl (=3-Methylbutyl), Isopropoxy, 2-Methylpropoxy und 3-Methylbutoxy.

In den Resten R¹ und/oder R² können ein, zwei, mehrere oder alle H-Atome durch Fluor substituiert sein.

Falls n = 0 ist, bedeutet R² vorzugsweise R-O-, R-COO- oder R-O-CO-O-, worin R geradkettiges oder verzweigtes Alkyl mit 1 bis 25 C-Atomen, in dem ein oder mehrere H-Atome durch Halogen substituiert sein können, und/oder worin eine oder mehrere nicht benachbarte -CH₂-Gruppen unabhängig voneinander durch -CO-, -O-CO-, -S-CO-, -CO-O-, -CO-S-, -O-, -S-, -CH=CH-, -CH=CF-, -CF=CH-, -CF=CF-, -C≡C-, -NH- oder -N(CH₃)- derart ersetzt sein können, dass keine Heteroatome benachbart zueinander stehen, bedeutet. R weist hierin vorzugsweise eine der zuvor für R¹ und/oder R² als bevorzugt angegebenen Bedeutungen auf.

Falls n = 1 oder 2 ist, bedeutet Z² vorzugsweise -O-CO-, -CO-O-, -CH₂-O- oder -CF₂-O-.

Von den Verbindungen sind diejenigen bevorzugt, in denen -(Z¹-A¹)ₘ- und/oder -(A²-Z²)ₙ- unabhängig voneinander eine Gruppe mit einem oder zwei sechsgliedrigen Ringen bedeutet.

In den Verbindungen, die beide Gruppen -(Z¹-A¹)ₘ- und -(A²-Z²)ₙ- aufweisen, können diese identisch oder verschieden sein. Besonders bevorzugt sind hierbei die Verbindungen, in denen diese beiden Gruppen verschieden sind.

Bevorzugte Teilformeln für die Gruppen -(Z¹-A¹)ₘ- und/oder -(A²-Z²)ₙ- sind nachfolgend aufgeführt, wobei auch die spiegelbildlichen Teilformeln hiervon bevorzugt sind. Zur Vereinfachung werden folgende Bezeichnungen eingeführt: Phe ist 1,4-Phenylen, PheL ist 1,4-Phenylen, das durch 1 bis 4 Substituenten L substituiert ist, worin L F bedeutet, und Cyc ist 1,4-Cyclohexylen.

| | |
|---|---|
| -Phe-Z- | T-1 |
| -Cyc-Z- | T-2 |
| -PheL-Z- | T-3 |
| -Phe-Z-Phe-Z- | T-4 |
| -Phe-Z-Cyc-Z- | T-5 |
| -Cyc-Z-Cyc-Z- | T-6 |
| -PheL-Z-Phe-Z- | T-7 |
| -PheL-Z-Cyc-Z- | T-8 |
| -PheL-Z-PheL-Z- | T-9 |

Besonders bevorzugt sind die Teilformeln T-1, T-3, T-7 und T-8.

In diesen Teilformeln hat Z eine der für Z¹ angegebenen Bedeutungen. Für den Fall -(A²-Z²)ₙ- kann Z auch eine der für Z² angegebenen Bedeutungen besitzen und L auch Chlor bedeuten.

Besonders bevorzugt bedeuten -( Z¹-A¹)ₘ- und/oder -( A²-Z²)ₙ- unabhängig voneinander eine der folgenden Teilformeln und ihre spiegelbildlichen Teilformeln hierzu, die über die Brücke Z mit der Tolan-Gruppe verbunden sind: 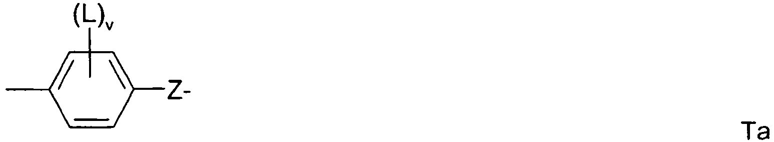 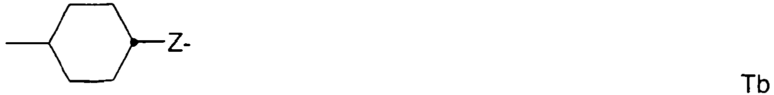 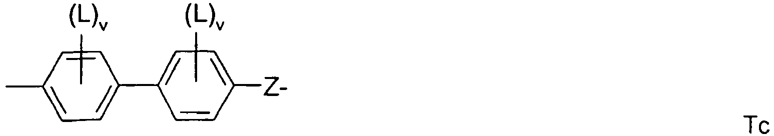 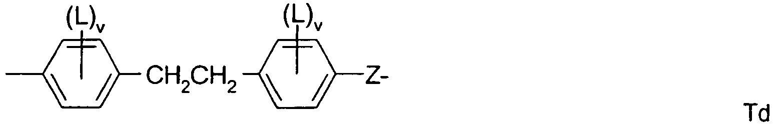 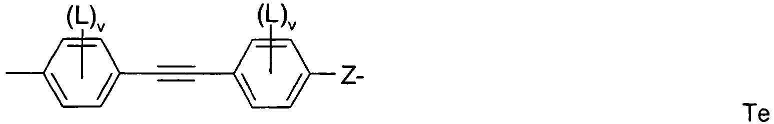 und im Fall -(Z²-A²)ₙ- auch: 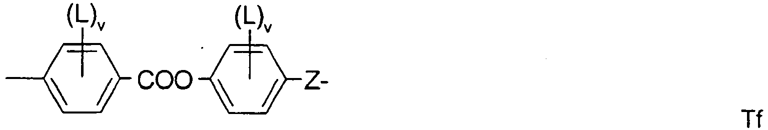 worin Z und L jeweils eine der zuvor angegebenen Bedeutungen aufweisen und v 0, 1, 2, 3 oder 4, vorzugsweise 0, 1 oder 2 ist.

Die Gruppe 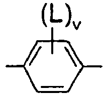 in den obigen Teilformeln und/oder die Gruppen 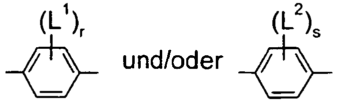 in den Formel I, II, III, IV, V, IX, X bedeuten vorzugsweise 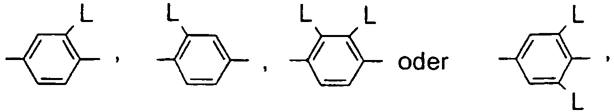 mit den zuvor für L¹, L² und L angegebenen Bedeutungen, wobei L¹, L² und L besonders bevorzugt F oder Cl bedeuten.

Besonders bevorzugt sind die Verbindungen, die
mindestens eine Gruppe 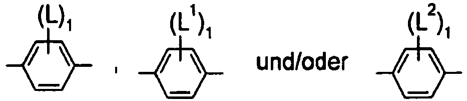 enthalten.

Ferner sind die Verbindungen bevorzugt, die
mindestens zwei Gruppen 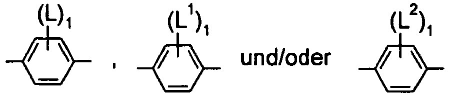 und/oder
mindestens eine Gruppe 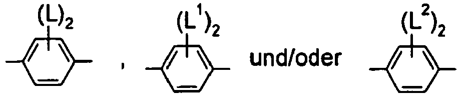 enthalten.

Die nachfolgenden, bevorzugten Ausführungsformen beziehen sich auf die polymerisierbaren Tolane der Formel X.

Die Gruppe P in der Formel X ist vorzugsweise eine Acrylat-, Methacrylat-, Vinyl-, Vinyloxy-, Epoxy-, Styryl- oder Propenylether-Gruppe.

Als Spacer-Gruppe Sp in den Verbindungen der Formel X können alle Gruppen eingesetzt werden, die dem Fachmann für diesen Zweck bekannt sind. Sp ist vorzugsweise eine lineare oder verzweigte Alkylen-Gruppe mit 1 bis 20 C-Atomen, besonders bevorzugt mit 1 bis 12 C-Atomen, in der eine oder mehrere nicht benachbarte CH₂-Gruppen durch -O-, -S-, -NH-, -N(CH₃)-, -CO-, -O-CO-, -S-CO-, -O-COO-, -CO-S-, -CO-O-, -CH(Halogen)-, -CH(CN)-, -CH=CH- oder -C≡C- ersetzt sein können.

Übliche Spacer-Gruppen sind zum Beispiel -(CH₂)ₒ-, -(CH₂CH₂O)ₚ-CH₂CH₂-, -CH₂CH₂-S-CH₂CH₂- oder -CH₂CH₂-NH-CH₂CH₂-, mit o = 2 bis 12 und p = 1 bis 3.

Bevorzugte Spacer-Gruppen sind Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen oder Butenylen.

Besonders bevorzugt sind die Verbindungen der Formel X, in denen Sp eine Alkyl- oder Alkoxy-Gruppe mit 2 bis 8 C-Atomen bedeutet. Hierbei sind geradkettige Gruppen besonders bevorzugt.

Gemäß einer weiteren Ausführungsform sind chirale Verbindungen der Formel X bevorzugt, die mindestens eine Spacer-Gruppe Sp umfassen, die eine chirale Gruppe der Formel XIII ist 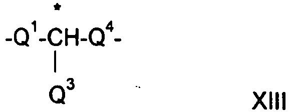 worin
Q¹ und Q³ jeweils eine der in bezug auf Formel XI angegebenen Bedeutungen aufweisen und
Q⁴ eine Alkylen- oder Alkylenoxy-Gruppe mit 1 bis 10 C-Atomen oder eine Einfachbindung und verschieden von Q¹ ist.

In dem Fall, dass R¹ P-Sp-X bedeutet, können die beiden Spacer-Gruppen Sp in den Verbindungen der Formel X gleich oder verschieden sein.

Von den zuvor als bevorzugt bezeichneten Verbindungen der Formel X sind diejenigen besonders bevorzugt, in denen p = 1 ist.

Ferner sind diejenigen Verbindungen bevorzugt, die sowohl eine Gruppe P-(Sp-X)ₚ- mit p = 0 als auch eine Gruppe P-(Sp-X)p- mit p = 1 aufweisen.

Sowohl die flüssigkristallinen Tolane der Formel IX als auch die polymerisierbaren, flüssigkristallinen Tolane der Formel X können gemäß oder analog bekannter Verfahren, wie sie beispielsweise in dem genannten Standardwerk "Methoden der Organischen Chemie" beschrieben sind, erhalten werden.

Vorzugsweise werden die Verbindungen der Formel IX und X aus den Tolanen der Formel I synthetisiert.

Das folgende Reaktionsschema 3 erläutert die Verwendung von Tolanen der Formel I zur Synthese von polymerisierbaren, flüssigkristallinen Tolanen der Formel X am Beispiel einer Verbindung der Formel X, in der n = 1 ist und Z² -CO-O- bedeutet.

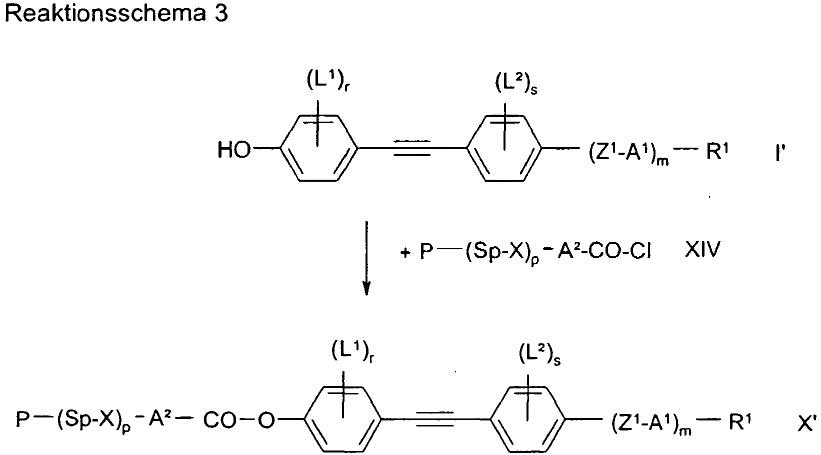

Das Tolan mit freier Hydroxy-Gruppe der Formel I' wird mit dem Säurechlorid der Formel XIV, das aus der entsprechenden Carbonsäure durch Umsetzung mit Thionylchlorid zugänglich ist, zu dem polymerisierbaren Tolan der Formel X' umgesetzt.

Das folgende Reaktionsschema 4 veranschaulicht die Verwendung von Tolanen der Formel I zur Synthese von flüssigkristallinen Tolanen der Formel IX am Beispiel einer Verbindung der Formel IX', in der n = 1 ist und Z² -CO-O- bedeutet.

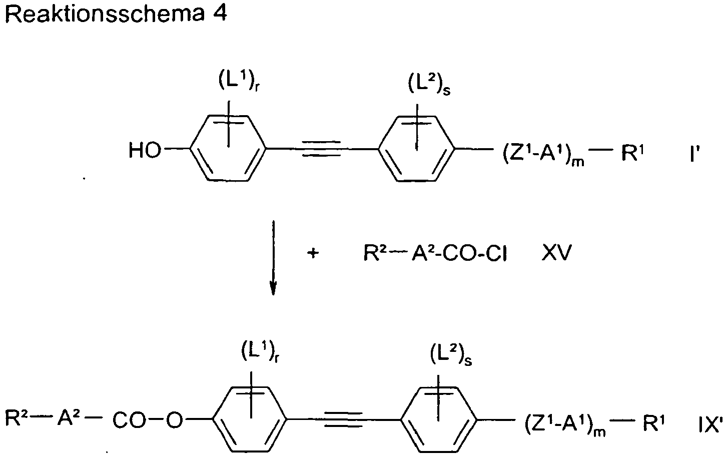

Die Synthese von flüssigkristallinen Tolanen der Formel IX wird am Beispiel einer Verbindung der Formel IX", in der n = 0 ist und R² R-CO-O-bedeutet, durch das folgende Reaktionsschema 5 verdeutlicht.

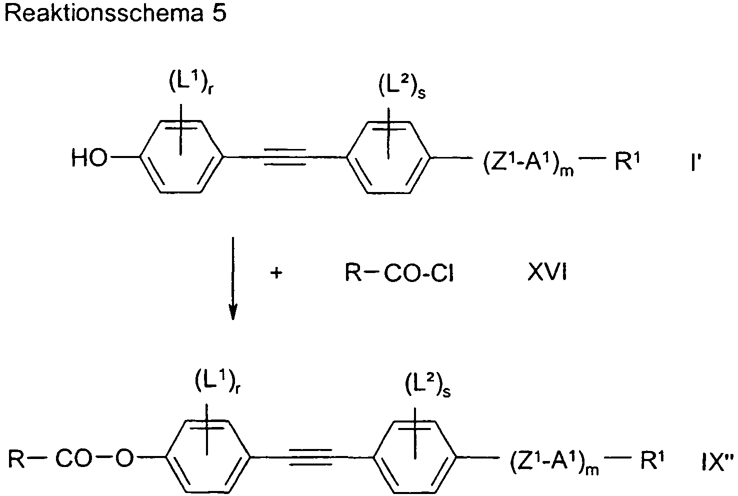

Die Synthese weiterer Tolane der Formel IX und X kann der Fachmann in Abwandlung der obigen Reaktionsschemata und unter Verwendung allgemein bekannter Verfahren ohne weiteres durchführen.

Ferner gibt es flüssigkristalline Medien mit mindestens zwei flüssigkristallinen Komponenten, die mindestens ein Tolan der Formel IX mit einer chiralen Gruppe enthalten.

Die Art der einzusetzenden Komponenten sowie deren Massenanteile richten sich nach der jeweilig angestrebten Verwendung. Nur beispielhaft sei hier auf die Ausführungen bezüglich der üblicherweise in solchen Medien verwendeten Zusammensetzungen in den Offenlegungsschriften WO 88/07514 A1 und WO 92/18448 A1 hingewiesen.

Je nach dem verwendeten Tolan und den übrigen Komponenten können solche Medien in elektrooptischen Anzeigeelementen verwendet werden, die nach dem Prinzip der verdrillten Zelle, dem Guest-Host-Effekt, dem Effekt der Deformation aufgerichteter Phasen oder dem Effekt der dynamischen Streuung beruhen. Solche Medien können auch in Displays Verwendung finden, die den ECB-Effekt nutzen. Übliche Anzeigeelemente nach dem Prinzip der verdrillten Zelle sind beispielsweise TN- und STN-Displays sowie solche mit einer aktiven Matrix, wie z.B. TFT-TN-Displays.

Die Erfindung wird im folgenden anhand von Ausführungsbeispielen näher erläutert.

### Beispiele

### Beispiel 1

### Herstellung von 4-n-Propyl-4'-hydroxytolan

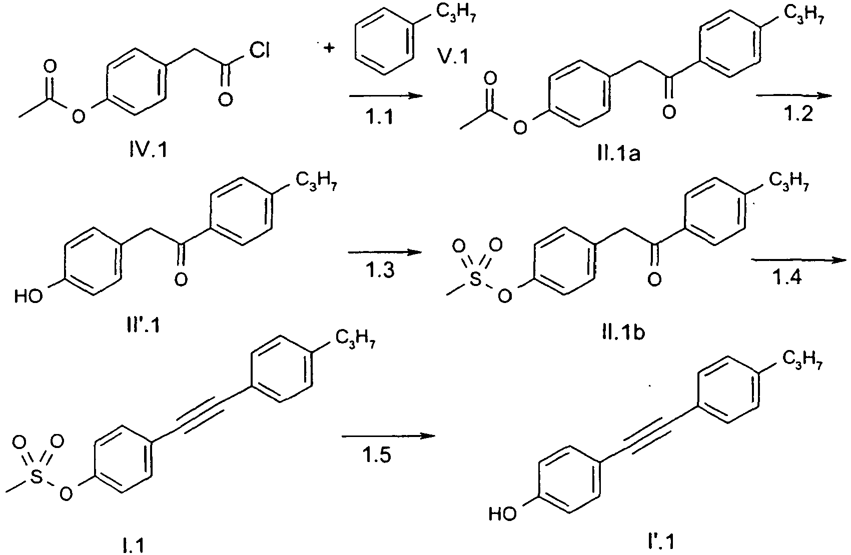

### 1.1 Herstellung von 1-(4-n-Propylphenyl)-2-(4-acetoxyphenyl)ethanon der Formel II.1a

100 g (4-Acetoxyphenyl)-essigsäurechlorid (IV.1) (hergestellt nach Walpole et al., J. Med. Chem. 36, 2362 (1993)) und 56 g 4-n-Propylbenzol (V.1) werden in 250 ml Dichlormethan gelöst. Diese Lösung wird unter Rühren und Kühlen zu einem Gemisch aus 125,3 g AlCl₃ in 500 ml Dichlormethan gegeben. Die Reaktionstemperatur soll dabei 10°C nicht überschreiten. Nachdem die Zugabe beendet ist, wird 4 Stunden bei 15°C gerührt. Dann gibt man das Reaktionsgemisch zu 260 ml Salzsäure (37%), die mit 500 ml Wasser verdünnt ist. Man rührt 15 Minuten und trennt dann die organische Phase ab. Die organische Phase wird zweimal mit je 250 ml Wasser gewaschen und dann zum Rückstand eingeengt. Der Rückstand wird aus einem geeigneten Lösungsmittel, wie Toluol oder Methyl-tert.-butylether, umkristallisiert.

### 1.2 Herstellung von 1-(4-n-Propylphenyl)-2-(4-hydroxyphenyl)ethanon der Formel II'.1:

115 g 1-(4-n-Propylphenyl)-2-(4-acetoxyphenyl)ethanon (II.1a) werden in 300 g Aceton gelöst. Diese Lösung gibt man langsam zu einer gut gerührten und auf 40°C erwärmten Lösung von 85 g NaOH in 200 g Methanol. Nachdem die Zugabe beendet ist, rührt man 1,5 Stunden bei 40°C. Dann lässt man unter Kühlen und Rühren das Reaktionsgemisch zu einem Gemisch aus 100 g HCl (37%) und 400 g Eis laufen. Dabei fallen hellbraune Kristalle aus. Diese werden isoliert, mit Wasser gewaschen und getrocknet. Das Produkt wird aus Methyl-tert.-butylether umkristallisiert.

### 1.3 Herstellung von 1-(4-n-Propylphenyl)-2-(4-methansulfonyloxyphenyl)ethanon der Formel II.1b:

Es werden 87 g 1-(4-n-Propylphenyl)-2-(4-hydroxyphenyl)ethanon (II'.1) in 200 ml Dichlormethan und 52 g Triethylamin vorgelegt. Unter Rühren und Kühlen gibt man dazu bei etwa 5°C bis 10°C 41,1 g Methansulfonylchlorid. Nachdem die Zugabe beendet ist, rührt man eine Stunde bei dieser Temperatur. Dann hydrolysiert man das Reaktionsgemisch mit 100 ml verdünnter HCl. Die wäßrige Phase wird abgetrennt und mit 100 ml Dichlormethan gewaschen. Die vereinigten Dichlormethanphasen werden mit NaHCO₃-Lösung (200 ml) und dann mit Wasser neutral gewaschen und eingedampft.

### 1.4 Herstellung von 4-n-Propyl-4'-methansulfonyloxytolan der Formel I.1:

110 g 1-(4-n-Propylphenyl)-2-(4-methansulfonyloxyphenyl)ethanon (II.1b) werden in 196 g Pyridin vorgelegt. Unter Rühren wird die Suspension auf 60°C erwärmt, dann gibt man 89 g Phosphoroxychlorid hinzu. Man rührt das Reaktionsgemisch 5 Stunden lang unter gelindem Sieden bei 110°C. Man läßt das Reaktionsgemisch auf etwa 80°C abkühlen und gibt es dann unter intensivem Rühren auf ein Gemisch aus 400 g Eis und 60 g HCl (37%). Die ausgefallenen Kristalle werden isoliert, gründlich mit Wasser gewaschen, unter Vakuum getrocknet und aus Methanol umkristallisiert.

### 1.5 Herstellung von 4-n-Propyl-4'-hydroxytolan der Formel I'.1:

69 g 4-n-Propyl-4'-methylsulfonyloxytolan (1.1) werden in 350 g Methanol suspendiert. Es wird 50 g Wasser und 55 g NaOH zugegeben. Das Reaktionsgemisch wird 2 Stunden unter Rühren auf 60°C erwärmt, dann läßt man die Lösung abkühlen. Man gibt sie ebenfalls unter kräftigem Rühren auf ein Gemisch aus 300 g Eis und 60 g HCl (37%). Die ausgefallenen Kristalle werden isoliert, mit Wasser gewaschen und im Vakuum getrocknet.

### Beispiel 2

### Herstellung von 4-n-Propyl-4'-hydroxytolan

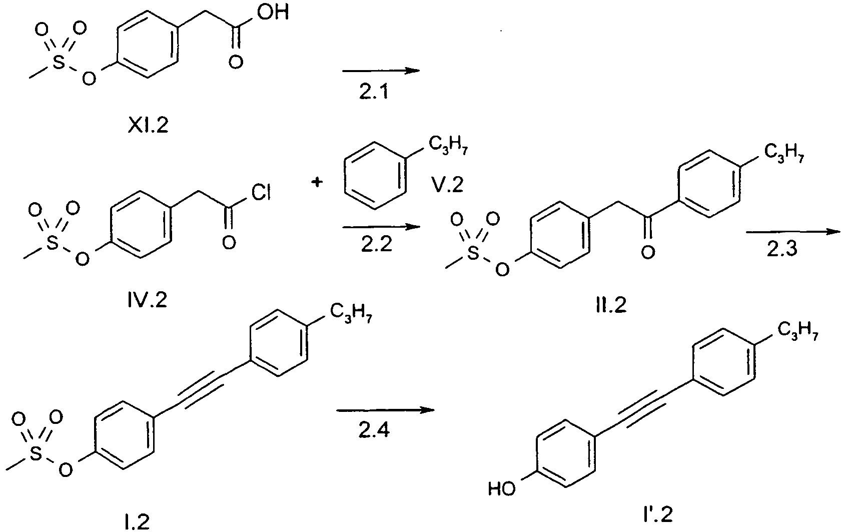

### 2.1 Herstellung von (4-Methansulfonyloxyphenyl)-essigsäurechlorid der Formel IV.2:

45 g (4-Methansulfonyloxyphenyl)-essigsäure (XI.2) (hergestellt gemäß Beispiel 1 der DE-OS 27 00 348) und 42 g Thionylchlorid werden gemischt. Das Gemisch erwärmt man vorsichtig auf etwa 40°C bis die Gasentwicklung aufhört. Dann wird das überschüssige Thionylchlorid im Vakuum abdestilliert. Der Rückstand wird ohne weitere Aufarbeitung direkt weiterverwendet.

### 2.2 Herstellung von 1-(4-n-Propylphenyl)-2-(4-methansulfonyloxyphenyl)ethanon der Formel II.2:

49 g (4-Methansulfonyloxyphenyl)-essigsäurechlorid (IV.2) und 26 g n-Propylbenzol (V.2) werden in 200 ml Dichlormethan gelöst. Diese Lösung trägt man langsam unter Temperaturkontrolle und Rühren bei maximal 10°C in eine Suspension von 58 g AlCl₃ in 150 ml Dichlormethan ein. Nachdem die Zugabe beendet ist, rührt man drei Stunden unter langsamem Erwärmen auf Raumtemperatur. Das Reaktionsgemisch wird in 1 I eiskalte, verdünnte Salzsäure eingetragen. Man rührt 15 Minuten und trennt dann die Phasen. Die wäßrige Phase wird mit 200 ml Dichlormethan extrahiert. Die vereinigten Dichlormethanphasen werden mit NaHCO₃-Lösung und mit Wasser neutral gewaschen, mit Na₂SO₄ getrocknet und zum Rückstand eingedampft.

### 2.3 Herstellung von 4-n-Propyl-4'-methansulfonyloxytolan der Formel 1.2:

Die Umsetzung erfolgt unter Verwendung des zuvor erhaltenen 1-(4-n-Propylphenyl)-2-(4-methansulphonyloxyphenyl)ethanon der Formel II.2 analog Beispiel 1.4.

### 2.4 Herstellung von 4-n-Propyl-4'-hydroxytolan der Formel I'.2:

Die Durchführung erfolgt unter Verwendung des zuvor erhaltenen 4-n-Propyl-4'-methansulfonyloxytolan der Formel I.2 analog Beispiel 1.5.

### Beispiel 3

### Herstellung von trans-4-n-Propylcyclohexyl-4'-hydroxytolan

Diese Verbindung erhält man analog Beispiel 2, wenn anstelle von n-Propylbenzol (V.2) trans-n-Propylcyclohexylbenzol eingesetzt wird.

### Beispiel 4

### Herstellung von 4-Pentylphenylethinyl-4'-phenoxycarbonyl-4"-phenoxypropylacrylat als polymerisierbares, flüssigkristallines Tolan

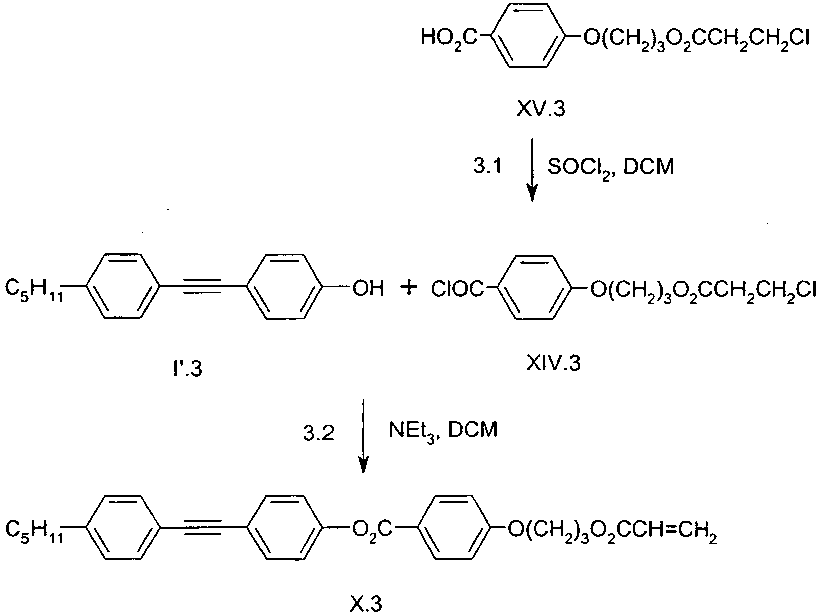

### 4.1 Herstellung von 4-(Oxypropyl-3-chloropropanoyl)benzoylchlorid der Formel XIV.3

5,0 g 4-(Oxypropyl-3-chloropropanoyl)benzoesäure der Formel XV.3 (17,4 mmol), 1,65 ml Thionylchlorid (22,6 mmol) und 3 Tropfen N-Methylpyrrolidon werden 3 Stunden in Dichlormethan (DCM) unter Rückfluss erhitzt. Die Reaktionslösung wird abgekühlt und zu einem farblosen Öl eingeengt. Es werden 5,4 g, entsprechend 100 % Ausbeute, erhalten, die direkt weiter verwendet werden.

### 4.2 4-Pentylphenyl-ethinyl-4'-phenoxycarbonyl-4"-phenoxypropyl-acrylat der Formel X.3

5,3 g des Säurechlorids der Formel XIV.3 (17,4 mmol), 4,6 g des Hydroxytolans der Formel I'.3 (17,4 mmol), das analog zu Beispiel 1 oder 2 erhältlich ist, und 14,6 ml Triethylamin (104,4 mmol) werden in 150 ml Dichlormethan (DCM) über Nacht bei 35°C gerührt. Die Mischung wird auf Raumtemperatur abgekühlt, mit verdünnter wäßriger Salzsäure und Wasser gewaschen und getrocknet (Na₂SO₄). Anschließend wird in einem Rotationsverdampfer bis zur Trockne eingeengt. Der Rückstand wird mittels Flash-Chromatographie gereinigt (Eluent: Dichlormethan:Petrolether 7:3), worauf 3,9 g eines weißen, glänzenden Feststoffs erhalten wird (Ausbeute: 86 %).

### Beispiel 5

### Herstellung von 1-(2-Methylbutyl)-benzol

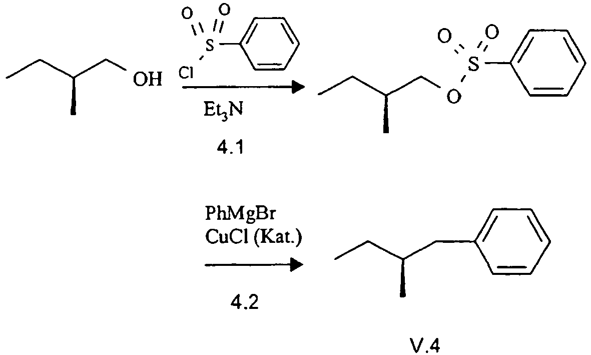

Die Synthese des chiralen 1-(2-Methylbutyl)-benzol der Formel V.4 erfolgt nach aus der Standardliteratur bekannten Verfahren ausgehend vom 2-Methyl-1-butanol durch Addition von Benzolsulfonsäurechlorid über den Benzolsulfonsäure-2-Methylbutylester, der mit der Grignard-Verbindung Phenylmagnesiumbromid in Gegenwart von Kupfer(I)-chlorid umgesetzt wird.

### Beispiel 6

### Herstellung von 1-(3,7-Dimethyloctyl)-benzol

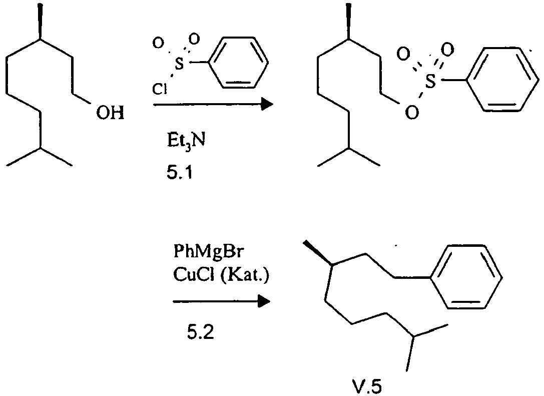

Die Synthese des chiralen 1-(3,7-Dimethyloctyl)-benzol der Formel V.5 erfolgt nach aus der Standardliteratur bekannten Verfahren ausgehend von dem optisch aktiven Dihydrocitronellol durch Addition von Benzolsulfonsäurechlorid über den entsprechenden Benzolsulfonsäureester, der mit der Grignard-Verbindung Phenylmagnesiumbromid in Gegenwart von Kupfer(I)-chlorid umgesetzt wird.

Die chiralen Arylverbindungen der Formel V, die in analoger Weise zu den Beispielen 5 und 6 zugänglich sind, dienen als Ausgangsverbindungen für die erfindungsgemäße Friedel-Crafts-Acylierung zur Synthese chiraler Benzylphenylketone der Formel II. Diese wiederum stellen chirale Synthesebausteine in der erfindungsgemäßen Tolan-Synthese dar. Über die auf diese Weise erhaltenen, erfindungsgemäßen chiralen Tolane der Formel I ist ein breites Spektrum an chiralen, flüssigkristallinen Tolanen der Formel IX sowie an chiralen, polymerisierbaren, flüssigkristallinen Tolanen der Formel X auf ökonomische Weise zugänglich.

### Beispiel 7

### Herstellung von 2,4-Dimethyl-4'-hydroxytolan

### 7.1 Herstellung von 1-(2,4-Dimethylphenyl)-2-(4-methansulfonyloxyphenyl)ethanon

460,4 g (2 mol) (4-Methansulfonyloxyphenyl)-essigsäure (XI.2) werden in 460 ml Acetonitril suspendiert und die Suspension auf 73°C (Innentemperatur) erwärmt. Innerhalb von 105 Minuten gibt man zu der gerührten Suspension 263 g (2,21 mol) Thionylchlorid, wobei die Temperatur durch leichtes Heizen zwischen 63°C und 71 °C gehalten wird (endotherm, Gasentwicklung!). Nachdem die Zugabe beendet ist, wird 1,5 Stunden bei 70°C bis 75°C nachgerührt (klare Lösung). Dann werden bei Normaldruck überschüssiges Thionylchlorid und Acetonitril abdestilliert bis eine Sumpftemperatur von 115°C erreicht ist. Der Rückstand wird mit 212,4 g m-Xylol (2 mol) versetzt und auf 40°C abgekühlt. Anschließend werden 500 ml Dichlormethan zugegeben.

In einer zweiten Apparatur werden 532 g (3,99 mol) Aluminiumchlorid in 750 ml Dichlormethan suspendiert und auf 0°C abgekühlt. Zu dieser gut gerührten Suspension gibt man langsam die Lösung des Säurechlorids in m-Xylol/Dichlormethan, wobei die Temperatur bei 0°C bis 5°C gehalten wird. Nachdem die Zugabe beendet ist, wird 1,5 Stunden bei 0°C bis 5°C nachgerührt. Diese Reaktionsmischung wird langsam in eine gut gerührte auf 40°C bis 42°C erhitzte Lösung von 37%iger Salzsäure in 1720 ml Wasser eingetropft. Dabei wird ein leichtes Vakuum angelegt und das Dichlormethan abdestilliert. Zum Schluß wird das Vakuum verstärkt und die letzten Reste von Dichlormethan werden abdestilliert. Dann kühlt man auf 10°C ab, saugt den Niederschlag ab und trocknet ihn. Man erhält 481 g Produkt.

### 7.2 Herstellung von 2,4-Dimethyl-4'-methansulfonyloxytolan.

356,6 g (1,12 mol) 1-(2,4-Dimethylphenyl)-2-(4-methan-sulfonyloxyphenyl)ethanon werden in 607 g (7,7 mol) Pyridin suspendiert und unter Rühren auf 60°C erwärmt. Es ergibt sich eine klare Lösung. Bei 60°C beginnt man langsam 206,1 g (1,344 mol) Phosphoroxychlorid zuzutropfen. Es wird eine Erwärmung bis 70°C zugelassen. Nachdem das Zutropfen beendet ist, erwärmt man langsam. Bei ca. 103°C steigt die Temperatur vergleichsweise schnell bis auf 109°C, obwohl das Heizen unterbrochen wird. Danach wird das Reaktionsgemisch bei 120°C Badtemperatur 2 Stunden nachgerührt, dann auf etwa 80°C abgekühlt und noch heiß in eine Mischung aus 1350 g Eis/Wasser und 106 ml 37%ige Salzsäure eingerührt. Es wird auf 15°C abgekühlt, der Niederschlag wird abgesaugt, mit Wasser gründlich gewaschen und getrocknet. Die Kristalle werden in 250 ml Dichlormethan aufgenommen und die Lösung wird über 400 g Kieselgel filtriert und mit Dichlormethan nachgewaschen. Nachdem das Lösungsmittel abdestilliert worden ist erhält man 273,7 g Produkt.

### 7.3 Herstellung von 2,4-Dimethyl-4'-hydroxytolan

Es werden 236 g Natronlauge (32%ig) in 140 ml Wasser vorgelegt und auf 85°C erwärmt. Man löst 270 g des hergestellten 2,4-Dimethyl-4'-methansulfonyloxytolans in 500 ml THF und tropft diese Lösung langsam bei etwa 70°C zur Natronlauge. Dabei destilliert das THF ab und man erhält eine Suspension. Zum gut gerührten Reaktionsgemisch gibt man 405 ml Wasser und rührt bei 95 bis 100°C (Badtemperatur). Es bildet sich zunächst ein dicker Brei, der sich aber langsam löst. Nach etwa 4 Stunden wird auf ca. 55°C abgekühlt und die Lösung in ein Gemisch aus 266 g Salzsäure und 970 g Eis eingerührt. Der Niederschlag wird abgesaugt, gründlich mit Wasser gewaschen und getrocknet. Man erhält 198,4 g Produkt.

### Beispiel 8

### Herstellung von 4-Hydroxy-3-chlor-4'-methoxytolan.

### 8.1 Herstellung von (3-Chlor-4-methansulfonyloxyphenyl)-essigsäure

Es werden in einer Rührwerksapparatur 750 ml Wasser und 1400 ml Natronlauge (32%) vorgelegt. Die Lösung wird auf 0°C bis 5°C abgekühlt und unter Rühren langsam 1800 g (3-Chlor-4-hydroxyphenyl)-essigsäure eingetragen. Dabei kann die Temperatur bis auf 30°C ansteigen. Die Lösung wird anschließend auf 0°C bis 15°C abgekühlt. Zu der gekühlten Lösung werden langsam 1000 ml Methansulfonsäurechlorid gegeben. Dabei hält man den pH-Wert der Reaktionslösung durch Zugabe von verdünnter NaOH (hergestellt aus 4500 ml Wasser und 1800 ml Natronlauge) bei Werten zwischen 11 und 11,5. Nachdem die Zugabe des Methansulfonylchlorids beendet ist, rührt man so lange bei pH 11 bis 11,5 nach, bis der pH-Wert ohne Zugabe von Natronlauge konstant bleibt. Anschließend gibt man unter Kühlung langsam weitere 300 ml Methansulfonylchlorid zu und hält wie oben beschrieben durch Zugabe verdünnter Natronlauge den pH-Wert konstant bei 11 bis 11,5. Nachdem die Zugabe des Methansulfonylchlorids beendet ist, rührt man so lange bei pH 11 bis 11,5 nach, bis der pH-Wert ohne Zugabe von Natronlauge konstant bleibt. Zu dem Reaktionsgemisch gibt man langsam eine Lösung von 3200 ml Wasser und 900 ml Eisessig. Dabei fällt das Produkt aus. Es wird filtriert und auf dem Filter gründlich mit Wasser gewaschen. Man erhält nach dem Trocknen (50°C, Vakuum) 2322 g Produkt.

### 8.2 Herstellung von 1-(4-Methoxyphenyl)-2-(3-chlor-4-methansulfonyloxyphenyl)ethanon

3000 g (3-Chlor-4-methansulfonyloxyphenyl)-essigsäure werden in 3000 ml Acetonitril suspendiert und auf 50°C erwärmt. Dazu gibt man unter kräftigem Rühren langsam 900 ml Thionylchlorid, wobei die Temperatur durch leichtes Heizen bei 62 bis 75°C gehalten wird. Dabei findet Gasentwicklung statt und es entsteht eine klare Lösung. Es wird bei 70 bis 75°C zwei Stunden nachgerührt. Überschüssiges Thionylchlorid und Acetonitril wird im Vakuum abdestilliert. Der Rückstand wird mit 1239 ml Anisol versetzt und auf 25°C abgekühlt, dann werden 5000 ml Dichlormethan zugegeben.

In einer zweiten Apparatur werden 3025 g Aluminiumchlorid in 5000 ml Dichlormethan suspendiert. Zu dieser auf 10°C gekühlten Suspension gibt man innerhalb von zwei Stunden unter Temperaturkontrolle bei 10°C bis 15°C langsam die Lösung des Säurechlorids in Anisol/Dichlormethan aus der ersten Apparatur zu. Nachdem die Zugabe beendet ist, wird 2 Stunden bei 10°C bis 15°C nachgerührt. Die Reaktionslösung wird langsam in ein gut gerührtes Gemisch aus 7 I Essigester und 18 I Wasser bei 40°C bis 55°C eingetragen. Dabei destilliert das Dichlormethan bei leichtem Vakuum (400 mbar) ab. Anschließend wird bei 23°C nachgerührt, gegebenenfalls müssen noch 4 I Ethylacetat zugegeben werden. Das ausgefallene Produkt wird abgetrennt und durch mehrmaliges heißes Ausrühren mit Ethylacetat und dann mit Aceton und Abkühlen auf Raumtemperatur gereinigt. Nach dem Trocknen erhält man 2,5 kg Produkt.

### 8.3 Herstellung von 4-Methansulfonyloxy-3-chlor-4'-methoxytolan

3,618 kg 1-(4-Methoxyphenyl)-2-(3-chlor-4-methansulfonyloxyphenyl)-ethanon werden unter Rühren in 6,15 I Pyridin auf 70°C erwärmt. Innerhalb von zwei Stunden gibt man bei 66°C bis 70°C 2,02 kg Phosphoroxychlorid zu. Man erwärmt langsam zunächst auf 100°C Manteltemperatur und rührt dann bei 110°C Manteltemperatur 6 Stunden nach. Zu der Reaktionslösung gibt man bei 90°C bis 100°C eine Lösung von 1 I Eisessig in 13 I Wasser (Vorsicht, exotherm!). Dann kühlt man auf Raumtemperatur ab und isoliert die ausgefallenen Kristalle, die mit Wasser gründlich gewaschen werden. Man nimmt die Kristalle in 30 I heißem Aceton auf, filtriert die heiße Lösung über 3 kg Kieselgel und wäscht mit 20 I heißem Aceton nach. Die Acetonlösung wird auf ca. 10 kg eingeengt und auf 10°C gekühlt. Die Kristalle werden abgetrennt, in heißem Toluol aufgenommen, nochmals über Kieselgel filtriert, die Lösung wird eingeengt und dann gekühlt. Nach dem Trocknen erhält man 1,8 kg Produkt.

### 8.4 Herstellung von 4-Hydroxy-3-chlor-4'-methoxytolan

Zu einer gut gerührten Mischung aus 140 ml Wasser, 138 ml NaOH (32%) und 475 ml THF gibt man 238 g 4-Methansulfonyloxy-3-chlor-4'-methoxytolan. Das Reaktionsgemisch wird langsam zum Rückfluß erwärmt (ca. 65°C) und drei Stunden gerührt. Dann wird das THF abdestilliert, zur Suspension 400 ml Wasser gegeben und bei 36°C bis 43°C ein Gemisch aus 138 ml Salzsäure (37%) und 600 ml Wasser zugegeben. Unter Rühren wird auf 20°C abgekühlt, die Kristalle werden abgesaugt und gründlich mit Wasser gewaschen. Nach dem Trocknen erhält man 180 g Produkt.

## Patentansprüche

1. Verfahren zur Herstellung von Tolanen der allgemeinen Formel I 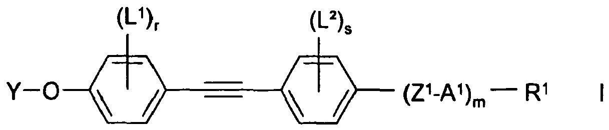 worin
Y eine Alkylsulfonyl-, Perfluoralkylsulfonyl-, Arylsulfonyl- oder Heteroarylsulfonyl- Schutzgruppe,
L¹, L² unabhängig voneinander, gleich oder verschieden F, Cl, eine Alkyl- und/oder Alkoxy-Gruppe mit 1 bis 6 C-Atomen, in der ein oder mehrere H-Atome durch Fluor substituiert sein können,
r, s unabhängig voneinander, gleich oder verschieden 0, 1, 2 oder 3,
Z¹ -C₂H₄-, -CH₂-CF₂-, -CF₂-CH₂-, -C₂F₄-, -CH₂-O-, -O-CH₂-, -CF₂-O-, -O-CF₂-, -C≡C-, -O-C(O)-, -C(O)-O- oder eine Einfachbindung,
A¹ 1,4-Phenylen, worin eine oder mehrere CH-Gruppen durch N ersetzt sein können, 1,4-Cyclohexylen, 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl, wobei diese Gruppen ein oder mehrfach mit F substituiert sein können,
m 0, 1 oder 2, und
R¹ H, Halogen, CN, geradkettiges oder verzweigtes Alkyl mit 1 bis 25 C-Atomen, worin ein oder mehrere H-Atome durch Fluor substituiert sein können, und/oder worin eine oder mehrere nicht benachbarte -CH₂-Gruppen unabhängig voneinander durch -O-, -C≡C- oder -CH=CH- ersetzt sein können, bedeuten
**dadurch gekennzeichnet, dass** man eine Verbindung der Formel II 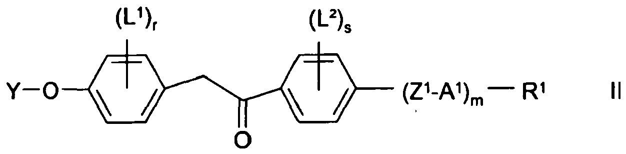 und/oder eine Verbindung der Formel III 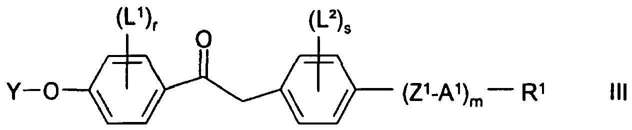 mit POCl₃ zusammen mit einer oder mehreren organischen Stickstoff-Basen umsetzt.

2. Verfahren gemäß Anspruch 1, **dadurch gekennzeichnet, dass** man als Base ein tertiäres Amin und/oder Pyridin einsetzt.

3. Verfahren gemäß Anspruch 1 oder 2, **dadurch gekennzeichnet, dass** Y eine Alkylsulfonyl-Schutzgruppe ist, in der die Alkylgruppe 1 bis 6 C-Atome aufweist, wobei in der Alkylgruppe ein oder mehrere H-Atome durch Fluor substituiert sein können.

4. Verfahren gemäß mindestens einem der Ansprüche 1 bis 3, **dadurch gekennzeichnet, dass** man vor der Durchführung des Verfahrensschritts gemäß Anspruch 1 die Verbindung der Formel II und/oder der Formel III, worin Y jeweils H bedeutet, mit einem organischen Sulfonylhalogenid und/oder Sulfonylanhydrid zu der entsprechenden Verbindung der Formel II und/oder III, worin Y jeweils eine Sulfonyl-Schutzgruppe bedeutet, umsetzt.

5. Verfahren gemäß mindestens einem der Ansprüche 1 bis 4, **dadurch gekennzeichnet, dass** man die Verbindung der Formel II durch Friedel-Crafts-Acylierung einer Verbindung der Formel IV 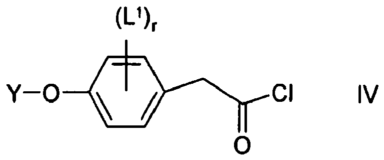 worin Y, L¹ und r die in Bezug auf Formel I in Anspruch 1 angegebenen Bedeutungen aufweisen, mit einer Verbindung der Formel V 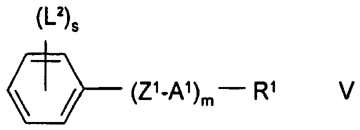 worin Z¹, A¹, R¹, m, L² und s die in Bezug auf Formel I in Anspruch 1 angegebenen Bedeutungen aufweisen, in Gegenwart eines Friedel-Crafts-Katalysators erhält.

6. Verfahren gemäß Anspruch 5, **dadurch gekennzeichnet, dass** man als Friedel-Crafts-Katalysator AlCl₃ einsetzt.

7. Verfahren gemäß mindestens einem der Ansprüche 1 bis 6, **dadurch gekennzeichnet, dass** man ein Tolan der Formel I gemäß Anspruch 1 in ein entsprechendes Tolan der Formel I, in der Y = H bedeutet, überführt.

## Claims

1. Process for the preparation of tolans of the general formula I 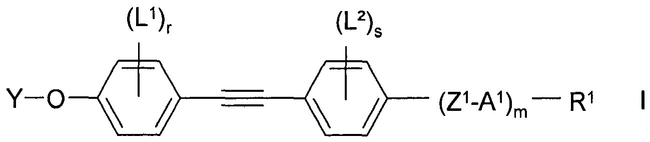 in which
Y denotes an alkylsulfonyl, perfluoroalkylsulfonyl, arylsulfonyl or heteroarylsulfonyl protecting group,
L¹, L², independently of one another, identically or differently, denote F, Cl, an alkyl and/or alkoxy group having 1 to 6 C atoms, in which one or more H atoms may be substituted by fluorine,
r, s, independently of one another, identically or differently, denote 0, 1, 2 or 3,
Z¹ denotes -C₂H₄-, -CH₂-CF₂-, -CF₂-CH₂-, -C₂F₄-, -CH₂-O-, -O-CH₂-, -CF₂-O-, -O-CF₂-, -C≡C-, -O-C(O)-, -C(O)-O- or a single bond,
A¹ denotes 1,4-phenylene, in which one or more CH groups may be replaced by N, 1,4-cyclohexylene, 1,4-cyclohexenylene, 1,4-bicyclo[2.2.2]octylene, piperidine-1,4-diyl, naphthalene-2,6-diyl, decahydronaphthalene-2,6-diyl or 1,2,3,4-tetrahydronaphthalene-2,6-diyl, where these groups may be mono- or polysubstituted by F,
m denotes 0, 1 or 2, and
R¹ denotes H, halogen, CN, or straight-chain or branched alkyl having 1 to 25 C atoms, in which one or more H atoms may be substituted by fluorine, and/or in which one or more non-adjacent -CH₂- groups may be replaced, independently of one another, by -O-, -C≡C- or -CH=CH-,
**characterised in that** a compound of the formula II 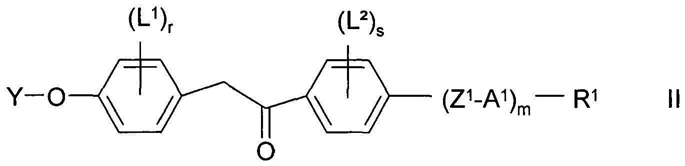 and/or a compound of the formula III 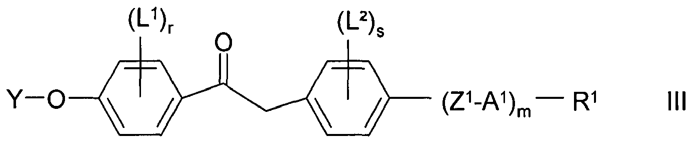 are reacted with POCl₃ together with one or more organic nitrogen bases.

2. Process according to Claim 1, **characterised in that** the base employed is a tertiary amine and/or pyridine.

3. Process according to Claim 1 or 2, **characterised in that** Y is an alkylsulfonyl protecting group in which the alkyl group has 1 to 6 C atoms, where one or more H atoms in the alkyl group may be substituted by fluorine.

4. Process according to at least one of Claims 1 to 3, **characterised in that**, before the process step according to Claim 1 is carried out, the compound of the formula II and/or of the formula III in each of which Y denotes H is reacted with an organic sulfonyl halide and/or sulfonyl anhydride to give the corresponding compound of the formulae II and/or III in each of which Y denotes a sulfonyl protecting group.

5. Process according to at least one of Claims 1 to 4, **characterised in that** the compound of the formula II is obtained by Friedel-Crafts acylation of a compound of the formula IV 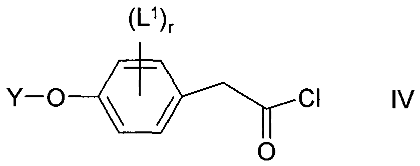 in which Y, L¹ and r have the meanings indicated in relation to formula I in Claim 1, using a compound of the formula V 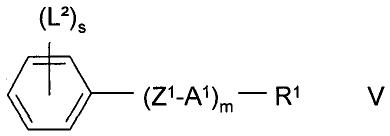 in which Z¹, A¹, R¹, m, L² and s have the meanings indicated in relation to formula I in Claim 1,
in the presence of a Friedel-Crafts catalyst.

6. Process according to Claim 5, **characterised in that** the Friedel-Crafts catalyst employed is AlCl₃.

7. Process according to at least one of Claims 1 to 6, **characterised in that** a tolan of the formula I according to Claim 1 is converted into a corresponding tolan of the formula I in which Y = H.

## Revendications

1. Procédé pour la préparation de tolanes de la formule générale I 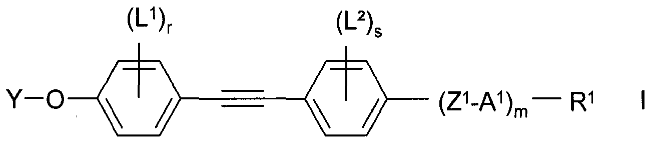 dans laquelle
Y représente un groupe de protection alkylsulfonyle, perfluoroalkylsulfonyle, arylsulfonyle ou hétéroarylsulfonyle,
L¹, L², représentent, indépendamment l'un de l'autre, de manière identique ou différente, F, CI, un groupe alkyle et/ou alcoxy comportant de 1 à 6 atomes de C, où un ou plusieurs atomes de H peut/peuvent être substitué(s) par fluor,
r, s, représentent, indépendamment l'un de l'autre, de manière identique ou différente, 0, 1, 2 ou 3,
Z¹ représente -C₂H₄-, -CH₂-CF₂-, -CF₂-CH₂-, -C₂F₄-, -CH₂-O-, -O-CH₂-, -CF₂-O-, -O-CF₂-, -C≡C-, -O-C(O)-, -C(O)-O- ou une liaison simple,
A¹ représente 1,4-phénylène, où un ou plusieurs groupes CH peut/peuvent être remplacé(s) par N, 1,4-cyclohexylène, 1,4-cyclohexénylène, 1,4-bicyclo[2.2.2]-octylène, pipéridine-1,4-diyle, naphtalène-2,6-diyle, décahydronaphtalène-2,6-diyle ou 1,2,3,4-tétrahydronaphtalène-2,6-diyle, où ces groupes peuvent être mono- ou polysubstitués par F,
m représente 0, 1 ou 2, et
R¹ représente H, halogène, CN, ou alkyle en chaîne droite ou ramifié comportant de 1 à 25 atomes de C, où un ou plusieurs atomes de H peut/peuvent être substitué(s) par fluor, et/ou où un ou plusieurs groupes -CH₂- non adjacents peut/peuvent être remplacé(s), indépendamment les uns des autres, par -O-, -C≡C- ou -CH=CH-,
**caractérisé en ce qu'**un composé de la formule II 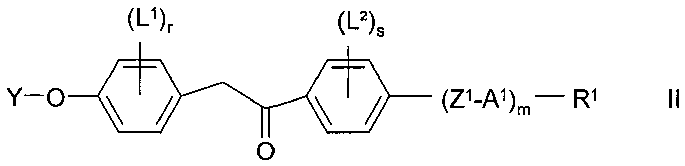 et/ou un composé de la formule III 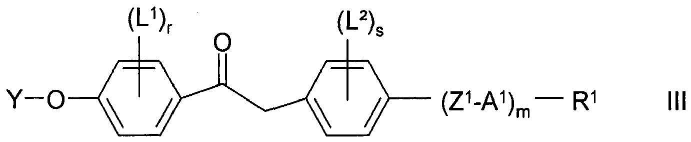 sont amenés à réagir avec POCl₃ en association avec une ou plusieurs bases azote organiques.

2. Procédé selon la revendication 1, **caractérisé en ce que** la base utilisée est un amine et/ou pyridine tertiaire.

3. Procédé selon la revendication 1 ou 2, **caractérisé en ce que** Y est un groupe de protection alkylsulfonyle où le groupe alkyle comporte de 1 à 6 atomes de C, où un ou plusieurs atomes de H dans le groupe alkyle peut/peuvent être substitué(s) par fluor.

4. Procédé selon au moins l'une des revendications 1 à 3, **caractérisé en ce que**, avant que l'étape de procédé selon la revendication 1 soit mise en oeuvre, le composé de la formule II et/ou de la formule III dans chacune desquelles Y représente H, est amené à réagir avec un halogénure de sulfonyle organique et/ou un anhydride de sulfonyle organique pour donner le composé correspondant des formules II et/ou III dans chacune desquelles Y représente un groupe de protection sulfonyle.

5. Procédé selon au moins l'une des revendications 1 à 4, **caractérisé en ce que** le composé de la formule II est obtenu par acylation de Friedel-Crafts d'un composé de la formule IV 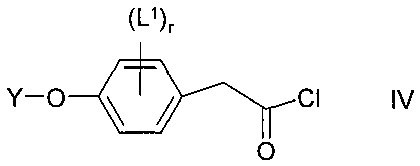 dans laquelle Y, L¹ et r présentent les significations indiquées en relation avec la formule I selon la revendication 1, en utilisant un composé de la formule V 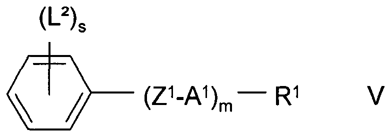 dans laquelle Z¹, A¹, R¹, m, L² et s présentent les significations indiquées en relation avec la formule I selon la revendication 1, en présence d'un catalyseur de Friedel-Crafts.

6. Procédé selon la revendication 5, **caractérisé en ce que** le catalyseur de Friedel-Crafts utilisé est ALCl₃.

7. Procédé selon au moins l'une des revendications 1 à 6, **caractérisé en ce qu'**un tolane de la formule I selon la revendication 1 est converti selon un tolane correspondant de la formule I dans laquelle Y = H.
